(19) Europäisches Patentamt
European Patent Office
Office européen des brevets

(11) EP 4 434 996 A1

(12) EUROPEAN PATENT APPLICATION

(43) Date of publication:
**25.09.2024 Bulletin 2024/39**

(21) Application number: **23305409.7**

(22) Date of filing: **24.03.2023**

(51) International Patent Classification (IPC):
***C07F 17/02*** *(2006.01)* ***A61P 35/00*** *(2006.01)*
***A61K 9/107*** *(2006.01)* ***A61K 41/00*** *(2020.01)*

(52) Cooperative Patent Classification (CPC):
**C07F 17/02; A61K 41/0042; A61K 41/0057; A61K 47/545; A61K 47/6907; A61P 35/00;**
A61K 9/1075

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Commissariat à l'Energie Atomique et aux Energies Alternatives**
  **75015 Paris (FR)**
- **Ustav Makromolekularni Chemie AV CR, V. V. I.**
  **16200 Praha 6 (CZ)**
- **Université Paris-Saclay**
  **91190 Gif-sur-Yvette (FR)**

(72) Inventors:
- **DORIS, Eric**
  **91191 GIF-SUR-YVETTE CEDEX (FR)**
- **GRAVEL, Edmond**
  **91191 GIF-SUR-YVETTE CEDEX (FR)**
- **HOANG, Stéphane**
  **91191 GIF-SUR-YVETTE (FR)**
- **PINNA, Guillaume**
  **92260 FONTENAY-AUX-ROSES (FR)**
- **VANDAMME, Marie**
  **92260 FONTENAY-AUX-ROSES (FR)**
- **HRUBÝ, Martin**
  **16206 PRAHA 6 (CZ)**

(74) Representative: **Cabinet Nony**
**11 rue Saint-Georges**
**75009 Paris (FR)**

# (54) MICELLES IN CANCER TREATMENT

(57) The invention relates to the pharmaceutical field.

In particular, the invention relates to the field of diagnosis and cancer treatment.

Herein, the inventors provide biocompatible and photodegradable micelle compositions that can target and release iron on demand, causing ferroptosis *in vivo,* and more particularly in cancer cells, and tissues thereof.

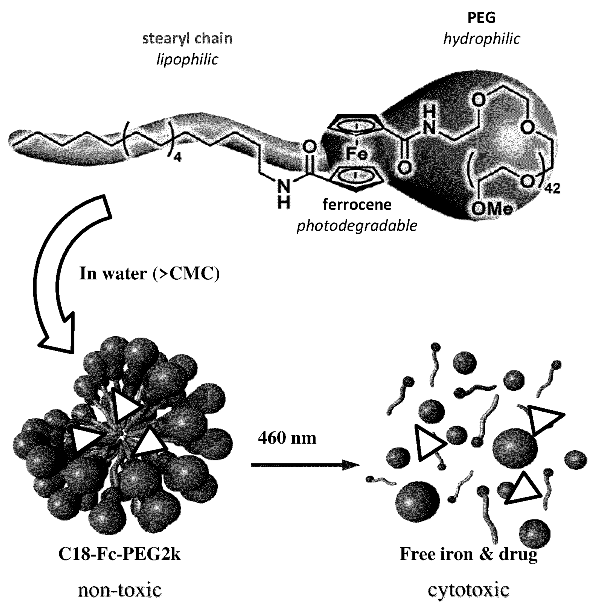


FIGURE 1

EP 4 434 996 A1

## Description

### TECHNICAL FIELD

The invention relates to the pharmaceutical field.

In particular, the invention relates to the field of diagnosis and cancer treatment.

### BACKGROUND OF THE INVENTION

Nanomedicine uses unique size-related properties of nanospecies, such as nanometric drug carriers, including species such as liposomes, dendrimers, polymers, nanoparticles, quantum dots and micelles. Such nanospecies are typically used for diagnosis and therapy of various diseases, including cancer.

Micelles are particularly advantageous for cancer applications thanks to their size that allows them to spontaneously accumulate in solid tumor tissues via the Enhanced Permeability and Retention (EPR) effect. This effect is caused by higher vascular permeability in such tissues, allowing local accumulation of the particles. As the lymphatic system is typically dysfunctional in malignant tissues, the particles are retained at the targeted location. Surface chemistry of nanomedicines can be tuned to fit the desired application. For instance, coating with poly(ethylene glycol) - PEGylation is known to limit opsonization, and thus, increase bloodstream residence time to achieve better passive accumulation. Different kinds of micelles were developed in the past decades to respond to various stimuli, such as changes in pH, redox potential, temperature, presence of light, ultrasound or enzymes.

Also photodynamic therapy (PDT) has been developed for decades to treat tumors. Indeed, the association of targeting nanoparticles coupled with a photosensitizer can generate reactive oxygen species (ROS) to induce cytotoxicity upon irradiation with light of appropriate wavelength. PDT has long proven its high efficiency for tumor treatment.

Apoptosis, necrosis and autophagy are the most common and best-known programmed cell death (PCD) mechanisms.

Anilkumar et al. ("Nanometric Micelles with Photo-Triggered Cytotoxicity"; Adv. Funct. Mater. 2014, 24, 5246-5252) teaches photo-activatable micelles with an approximate size of about 12 nm, using a nitrobenzyl derivatives as a photo-labile groups, to trigger cytotoxicity upon irradiation at 365 nm leading to cell death.

Mao et al. ("Delivery of Doxorubicin from Hyaluronic Acid-Modified Glutathione-Responsive Ferrocene Micelles for Combination Cancer Therapy"; Mol. Pharmaceutics 2019, 16, 987-994) teaches micelles with an approximate size of about 100 nm, formed by the self-assembly of a ferrocenium-tetradecyl (Fe-$C_{14}$) prodrug, in complex with hyaluronic acid, for encapsulating doxorubicin, and for combination cancer therapy.

Wei et al. ("Preparation of Novel Ferrocene-Based Shell Cross-Linked Thermoresponsive Hybrid Micelles with Antitumour Efficacy"; J. Phys.Chem. B 2010, 114, 5309-5314) teaches the assembly of an amphiphilic copolymer with a ferrocene derivative acting as a difunctional cross-linker. The resulting shell cross-linked assembly is characterized by an approximate size of about 120 nm, with cytotoxic properties.

Xiao et al. ("Amphiphilic block copolymers with aldehyde and ferrocene-functionalized hydrophobic block and their redox-responsive micelles"; J. Mater. Chem., 2010, 20, 8375-8381) teaches a redox stimulus-responsive amphiphilic, ferrocene-containing, block copolymer which can undergo self-assembly into micelles with size in the 96-153 nm range. It is proposed that such self-assembled micelles could allow the redox-controlled release of encapsulants.

Yet, there remains a need for developing biocompatible formulations, which can trigger cytotoxicity on demand in a controlled manner, and induce cell death in the tumor tissue while avoiding off-target damage of healthy tissues.

There also remains a need for developing biocompatible drug carriers, in particular inducible drug carriers, especially in the context of cancer therapy.

The invention has for purpose to meet the above-mentioned needs.

### SUMMARY OF THE INVENTION

According to a **first main embodiment,** the invention relates to a ferrocenyl-derivative compound, or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentandienyl ring is substituted with at least one **hydrophilic moiety,** said hydrophilic moiety being different from -OH, -C(=O)H or - C(=O)OH.

According to a **second main embodiment,** the invention relates to a biocompatible micelle composition, characterized in that the micelle comprises one or more ferrocenyl-derivative compound(s) according to the invention, or a pharmaceutically acceptable salt thereof.

According to a **third main embodiment,** the invention relates to a pharmaceutical composition comprising a biocompatible micelle according to the invention, or a ferrocenyl-derivative compound, or pharmaceutically acceptable salt thereof, according to the invention.

According to a **fourth main embodiment,** the invention relates to a kit comprising:

- one or more ferrocenyl-derivative compound(s), or pharmaceutically acceptable salt thereof, according to the invention, or a biocompatible micelle composition according to the invention;
- one or more photosensitizer(s) having an absorption band equal or superior to 620 nm.

According to a **fifth main embodiment,** the invention relates to a method for preparing a ferrocenyl-derivative compound, or pharmaceutically acceptable salt, according to the invention, comprising the steps of:

a) providing a precursor ferrocenyl-derivative compound comprising two cyclopentadienyl rings, each of the said rings being substituted with a reactive group;
b) bringing the precursor ferrocenyl compound derivative into contact with (i) a **hydrophobic moiety** consisting of an aliphatic chain-containing moiety, the aliphatic chain having at least 4 carbon atoms, and (ii) a **hydrophilic moiety,** said hydrophilic moiety being different from -OH, -C(=O)H or - C(=O)OH;
c) recovering the ferrocenyl-derivative compound.

According to a **sixth main embodiment,** the invention relates to a method for preparing a biocompatible micelle composition according to the invention, comprising a step of:

a) providing a composition comprising ferrocenyl-derivative compounds, or pharmaceutically acceptable salts thereof, according to the invention, and optionally a wavelength absorbing photosensitizer having an absorption band equal or superior to 620 nm;
b) ultra-sonicating the composition of step a);

thereby preparing the micelle composition.

## BRIEF DESCRIPTION OF THE FIGURES

**Fig. 1:** General scheme showing the different stages from the amphiphiles self-assembly into micelles to their cytotoxicity induced by blue light irradiation.
**Fig. 2:** Synthesis of both amphiphiles **C18-Fc-PEG2k (3)** and **C18-PEG2k (5).**
**Fig. 3: C18-Fc-PEG2k** (plain line) and **C18-PEG2k** (dashed line) **A)** DLS measurement shows a mean hydrodynamic diameter $D_h$ of 10 nm. **B)** Zeta potential measurement displays a neutral charge surface.
**Fig. 4:** Photodegradation of **C18-Fc-PEG2k** at Å = 460 nm. UV-vis spectra of before and after irradiation showing the disappearance of the ferrocene absorption band at 442 nm.
**Fig. 5:** XPS measurement of the amphiphile sample before irradiation and after irradiation (curve shifted to the left).
**Fig. 6:** Photodegradation of **C18-Fc-PEG2k** over 15 min. Complete photodegradation was achieved in 4 min.
**Fig. 7:** Irradiation of A) **NR@C18-Fc-PEG2k** and **B) NR@C18-PEG2k.** NR is stable under these conditions as shows the control experiment.
**Fig. 8:** Photodegradation of **A) C18-Fc-PEG2k** and **B) TPBC@C18-Fc-PEG2k** at Å = 740 nm up to 15 min.
**Fig. 9.** Plain lines: encapsulation of **TPBC, DPBF** and **TPBC+DPBF** in **C18-PEG2k** micelles. Dashed lines: **DPBF** and **TPBC** are insoluble in water. **B)** Irradiation of **DPBF@C18-PEG2k** at $\lambda$ =740 nm up to 240 sec. **C)** Irradiation of **TPBC-DPBF@C18-PEG2k** up to 20 min. **D)** Focus on the 400 nm region displaying the photo-oxidation of **TPBC** back to **TPP.**
**Fig. 1:** MCF-7 cells treated with **A) C18-Fc-PEG2k** and **B) C18-PEG2k** without (left column) and with (right column) irradiation at 460 nm for 10 min.

## DETAILED DESCRIPTION

Ferroptosis is a free iron-dependent programmed-cell death (PCD) that involves high level of lipid peroxides

leading to cell death, which is different from apoptosis, necrosis and autophagy. Lipid peroxides are naturally present in cells through polyunsaturated fatty acid (PUFA) peroxidation involving the Fenton reaction and lipoxygenases (LOXs). PUFA peroxides are unstable and can undergo radical addition with nearby PUFAs, damaging cell membranes. Also, ferroptosis is iron-dependent, meaning that increasing the intracellular free iron level leads to dysregulation of iron homeostasis, causing more lipid peroxidation.

Herein, the inventors provide biocompatible and photodegradable micelle compositions that can target and release iron on demand, causing ferroptosis *in vivo,* and more particularly in cancer cells, and tissues thereof.

The micelle compositions are characterized by the presence of one or more ferrocenyl-derivative compounds, the said compounds comprising two substituted cyclopentadienyl rings, one being substituted with a **hydrophobic moiety** and the other cyclopentandienyl ring being substituted with a **hydrophilic moiety.**

Surprisingly, such ferrocenyl-derivative compounds can behave as amphiphilic molecules as an activatable linker while retaining the ability to be incorporated, or self-assembled, into nanometric micelles: they can be photodecomposed at a specific irradiation wavelength, thus generating ferric hydroxides and organic by-products, thereby leading to an hydrolytically labile Fe(III) ferrocenium(+) species as an intermediate. This increase of intracellular iron can also be referred herein as *"iron overload".*

Without wishing to be bound by the theory, the inventors propose herein that the solvent also plays a role in the photodegradation process, and that the protic and nucleophilicity properties of the solvent are one reason why the photodegradation occurs the fastest in water and methanol.

In particular, it is proposed herein that the substitution of both cyclopentadienyl (Cp) rings in the ferrocene unit by acyl groups, as present for example in carbonyl (C=O)-substituted cyclopentadienyl rings, or by nitrogen-containing groups, as present for example in nitro ($NO_2$)-substituted cyclopentadienyl rings, or by sulfur-containing groups, as present for example in sulfonic acid ($SO_3H$)-substituted cyclopentadienyl rings, introduces strong metal-to-ligand charge transfer (MLCT), which then contributes to the fast photodecomposition of the ferrocene unit using visible light.

Accordingly, the inventors demonstrate herein that ferrocenyl-derivative compounds according to the invention **(i)** are stabilized in the form of micelles, **(ii)** can promote ferroptosis *in vivo* and **(iii)** are sensitive to an external stimulus.

In particular, the inventors demonstrate that ferrocenyl-derivative compounds according to the invention may self-assemble into nanometric micelles having an average size equal or inferior to 40 nm, which is compatible with efficient cell internalization, and which may also prevent immobilization *in vivo* by the extracellular collagen network, and thus permit their deep diffusion in the tumor tissue. Such self-assembled nanometric micelles may be characterized as in **figure 1;** with the hydrophobic part of the amphiphilic ferrocenyl-derivative compounds forming the core of the micelle, and the hydrophilic part of the compounds forming the shell.

Also, the inventors demonstrate that the micelle compositions are compatible with the encapsulation of a hydrophobic cargo. Its release can be synchronized with micelle photo-degradation. In particular, when bacteriochlorin is incorporated into such micelles, highly tissue-penetrable 740 nm near infrared light can be used to trigger micelle degradation by photodynamic effect mediated by singlet oxygen production. The micelles were tested on cell culture and showed efficient light-triggered ferroptosis.

Those properties thus render the micelle compositions according to the invention particularly suitable as a medicament, and more particularly for the treatment or prevention of solid tumors, or any other pharmaceutical use requiring high tissue penetration *in vivo.*

## Main embodiments

According to a **first main embodiment,** the invention relates to a ferrocenyl-derivative compound, or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentandienyl ring is substituted with at least one **hydrophilic moiety,** said hydrophilic moiety being different from -OH, -C(=O)H or - C(=O)OH.

According to a particular embodiment, the ferrocenyl-derivative compounds of the invention are characterized in that each cyclopentadienyl ring is selected from: a carbonyl (C=O)-substituted cyclopentadienyl ring, a nitro ($NO_2$)-substituted cyclopentadienyl ring, or a sulfonic acid ($SO_3H$)-substituted cyclopentadienyl ring.

According to some more particular embodiments, the ferrocenyl-derivative compounds of the invention are characterized in that each cyclopentadienyl ring is a carbonyl (C=O)-substituted cyclopentadienyl ring.

According to some more particular embodiments, the ferrocenyl-derivative compounds of the invention are

characterized in that each cyclopentadienyl ring is a nitro ($NO_2$)-substituted cyclopentadienyl ring.

According to some more particular embodiments, the ferrocenyl-derivative compounds of the invention are characterized in that each cyclopentadienyl ring is a sulfonic acid ($SO_3H$)-substituted cyclopentadienyl ring.

According to a particular embodiment, the ferrocenyl-derivative compounds of the invention are of formula (I) or (I'):

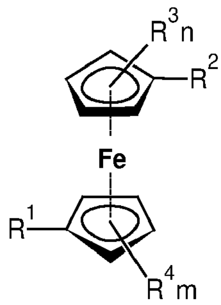
(I),
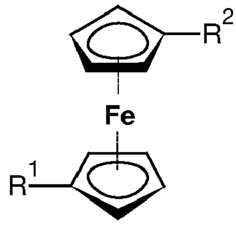
(I') ;

wherein:

n and m are identical or different, and equal to 0, 1, 2, 3 or 4;

$R^1$ is a **hydrophobic moiety** consisting of a substituted or unsubstituted, saturated or unsaturated, aliphatic $C_4$-$C_{36}$ chain-containing moiety;

$R^2$ is a **hydrophilic moiety,** said $R^2$ being different from -OH, -C(=O)H or - C(=O)OH;

$R^3$ and $R^4$ are identical or different, and selected from H, or optionally substituted acyl, alkyl, alkenyl, aryl, cycloalkyl, alkaryl, aralkyl, heteroaryl and heterocycloalkyl groups ; with the proviso that $R^3$ is not a substituted or unsubstituted, saturated or unsaturated, aliphatic $C_4$-$C_{36}$ chain-containing moiety.

According to particular embodiments of formula (I) or (I'), n and m are equal to 0 or 1; and most preferably n and m are equal to 0, and the ferrocenyl-derivative compounds are of formula (I').

When n and m are equal to 1, 2, 3 or 4, then $R^3$n and $R^4$m are preferably identical.

According to a more particular embodiment, ferrocenyl-derivative compounds of the invention are of formula (Ia) or (Ib) or (Ic) or (Id) or (Ie) or (If) or (Ig) or (Ih):

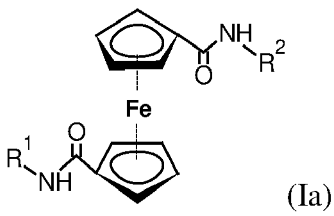
(Ia) ;
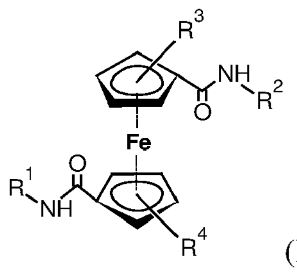
(Ib) ;

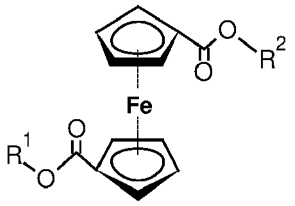
(Ic) ;
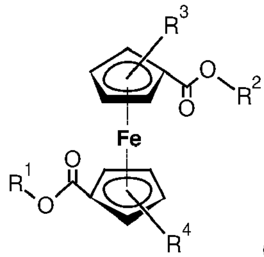
(Id) ;

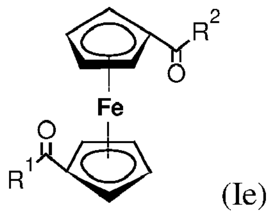 (Ie) ; 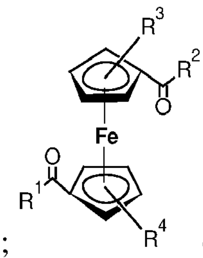 (If) ;

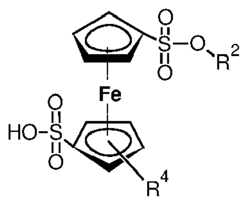 (Ig) ; 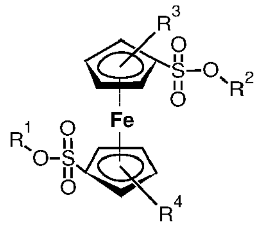 (Ih) ;

wherein $R^1$ is a **hydrophobic moiety** consisting of a substituted or unsubstituted, saturated or unsaturated, aliphatic $C_4$-$C_{36}$ chain-containing moiety;
$R^2$ is a **hydrophilic moiety;**
with the proviso that $R^2$ is not -H for formulae (Ic) and (Id);
with the proviso that $R^2$ is not -H nor -OH for formulae (Ie) and (If)
$R^3$ and $R^4$ are identical or different, and selected from H, or optionally substituted acyl, alkyl, alkenyl, aryl, cycloalkyl, alkaryl, aralkyl, heteroaryl and heterocycloalkyl groups; with the proviso that $R^3$ is not a substituted or unsubstituted, saturated or unsaturated, aliphatic $C_4$-$C_{36}$ chain-containing moiety.

According to said more particular embodiments, n and m are equal to 0 or 1; and most preferably n and m are equal to 0, and the ferrocenyl-derivative compounds are of formula (Ia) or (Ic) or (Ie) or (Ig).

When n and m are equal to 1, 2, 3 or 4, then $R^3$n and $R^4$m are preferably identical.

According to some embodiments, the ferrocenyl-derivative compounds of the invention, such as those selected from formulae (I) or (Ib) or (Id) or (If) or (Ih), are characterized in that at least one cyclopentadienyl ring, for example $R^3$ or $R^4$, is substituted with at least one moiety selected from the group consisting of:

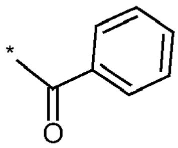 , 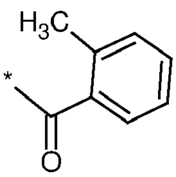 ; 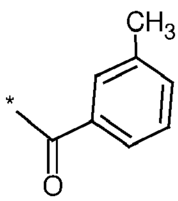 ; 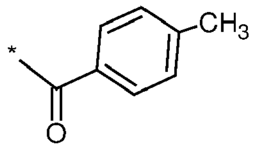 ; 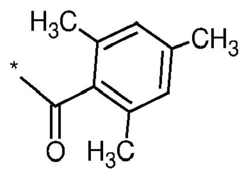 ;

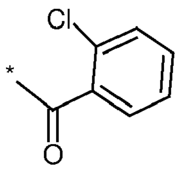 ; 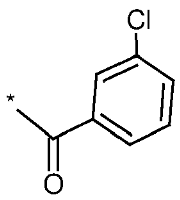 ; 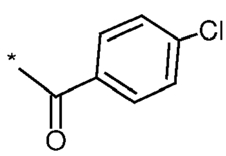 ; 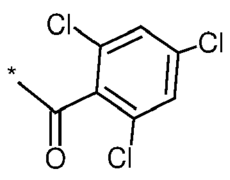 ; 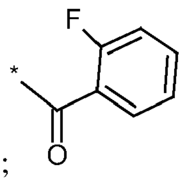 ;

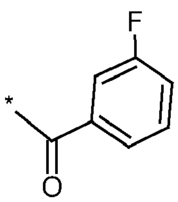 ; 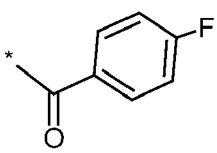 ; 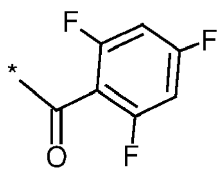 ; 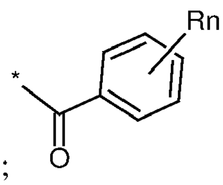 .

with R being selected from a group consisting of: a hydrogen atom, a halogen atom or a group chosen among a -CN group, a hydroxyl group, a (C1 C3)fluoroalkyl group, a (C1 C3)fluoroalkoxy group, a -$NO_2$ group, a (C1 C3)alkoxy group, a phenoxy group and a (C1-C3)alkyl group ;
with n being equal to 0, 1, 2 or 3.

According to the present invention, the ferrocenyl-derivative compounds are characterized in that the cyclopentandienyl ring which is substituted with at least one **hydrophilic moiety** is characterized in that said hydrophilic moiety is different from -OH, -C(=O)H or - C(=O)OH.

According to some embodiments, the ferrocenyl-derivative compounds are characterized in that the cyclopentandienyl ring which is substituted with at least one **hydrophilic moiety** is characterized in that said hydrophilic moiety is different from -OH, or -$C_x$(=O)H or - $C_x$(=O)OH, with x being equal to 1, 2 or 3.

According to some embodiments, the ferrocenyl-derivative compounds are characterized in that the cyclopentandienyl ring which is substituted with at least one **hydrophilic moiety** is characterized in that:

- said hydrophilic moiety is different from -OH, or -C(=O)H or - C(=O)OH;
- said hydrophilic moiety is selected from: an hydroxyl-containing moiety, a sulfate-containing moiety, a sulfonate-containing moiety, a carboxylate-containing moiety, a phosphate-containing moiety, an amine-containing moiety, a polyester-containing moiety, a polyether-containing moiety, a polyethylene glycol (PEG)-containing moiety.

According to some embodiments, the ferrocenyl-derivative compounds are characterized in that the cyclopentandienyl ring which is substituted with at least one **hydrophilic moiety** is characterized in that:

- said hydrophilic moiety is different from -OH, or -C(=O)H or - C(=O)OH;
- said hydrophilic moiety is an hydroxyl-containing moiety, for example an alcohol or a polyol.

According to some embodiments, the ferrocenyl-derivative compounds are characterized in that the cyclopentandienyl ring which is substituted with at least one **hydrophilic moiety** is characterized in that:

- said hydrophilic moiety is different from -OH, or -$C_x$(=O)H or - $C_x$(=O)OH, with x being equal to 1, 2 or 3;
- said hydrophilic moiety is selected from: an hydroxyl-containing moiety, a sulfate-containing moiety, a sulfonate-containing moiety, a carboxylate-containing moiety, a phosphate-containing moiety, an amine-containing moiety, a polyester-containing moiety, a polyether-containing moiety, a polyethylene glycol (PEG)-containing moiety.

According to some embodiments, the ferrocenyl-derivative compounds are characterized in that the cyclopentandienyl ring which is substituted with at least one **hydrophilic moiety** is characterized in that:

- said hydrophilic moiety is different from -OH, or -$C_x$(=O)H or - $C_x$(=O)OH, with x being equal to 1, 2 or 3;
- said hydrophilic moiety is an hydroxyl-containing moiety, for example an alcohol or a polyol.

According to some embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophilic moiety** is selected from a group consisting of: a hydroxyl-containing moiety, such as alcohols and/or polyols, polyoxyalkylenes, polyvinyl alcohols, polyvinyl-pyrrolidones, poly(2-methyl-2-oxazoline), a sulfate-containing moiety, a sulfonate-containing moiety, a carboxylate-containing moiety, a phosphate-containing moiety, an amine-containing moiety, a polyester-containing moiety, a polyether-containing moiety, a polyethylene glycol (PEG)-containing moiety.

According to some embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophilic moiety** is selected from a substituted or unsubstituted carboxylic acid, such as a $C_4$-$C_{20}$ or even $C_4$-$C_{12}$ carboxylic acid, or a substituted or unsubstituted fatty alcohol, such as a $C_4$-$C_{20}$ or even $C_4$-$C_{12}$ fatty alcohol.

According to some embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophilic moiety** is selected from a phospholipid, or polar head group thereof, in particular selected from the group consisting of: a phosphocholine-, choline-, phosphoethanolamine-, ethanolamine-, phosphoserine-, serine-, phosphoinositol-, inositol-, inositol-phosphate-, inositol-bisphosphate-, or inositol trisphosphate-containing moiety.

According to exemplified and preferred embodiments, the hydrophilic moiety consists of a polyethylene glycol (PEG) polymer or a polyethylene glycol (PEG) polymer-containing moiety; for example comprising from 4 to 200 ethoxy monomers, in particular comprising from 8 to 100 ethoxy monomers, preferably comprising from 30 to 60 ethoxy monomers.

In a non-limitative manner, a polyethylene glycol (PEG) polymer-containing moiety may thus comprise from 4 to 200 ethoxy momoners, which encompasses 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100, 110, 120, 130, 140, 150, 160, 170, 180, 190, 200 ethoxy monomers.

According to some embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophobic moiety** is a substituted or unsubstituted, saturated or unsaturated, aliphatic chain-containing moiety, the aliphatic chain having at least 4 carbon atom; the aliphatic chain having, for example, at least 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 carbon atoms.

According to some embodiments, the aliphatic chain may, for example, consist of a substituted or unsubstituted, saturated or unsaturated, aliphatic $C_4$-$C_{36}$ chain-containing moiety; in particular a linear, saturated or unsaturated, substituted or unsubstituted, aliphatic $C_4$-$C_{36}$ chain-containing moiety; for example a linear, saturated or unsaturated, substituted or unsubstituted, aliphatic $C_{12}$-$C_{36}$ chain-containing moiety.

According to some particular embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophobic moiety** consists of a substituted or unsubstituted, saturated or unsaturated, aliphatic $C_{12}$-$C_{36}$ chain-containing moiety.

According to some particular, non-mutually-exclusive embodiments, the ferrocenyl-derivative compounds of the invention, such as those selected from (I) or (I') or (Ia) or (Ib) or (Ic) or (Id) or (Ie) or (If) or (Ig) or (Ih), are characterized in that the **hydrophobic moiety** is fluorinated or perfluorinated.

According to some even more particular embodiments, the ferrocenyl-derivative compounds of the invention are of formula (IIa) or (IIb) or (IIc) or (IId) or (IIe) or (IIf) or (IIg) or (IIh) or (IIi) or (IIj) or (IIa) or (IIk) or (IIl) or (IIm):

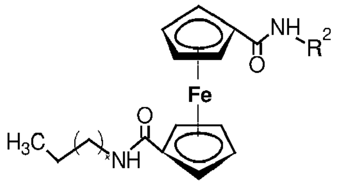 (IIa) ; 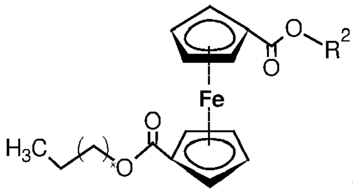 (IIb) ;

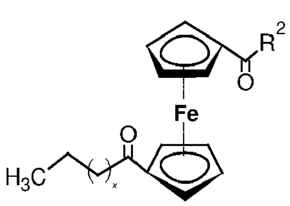 (IIc) ; 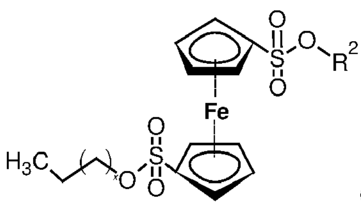 (IId) ;

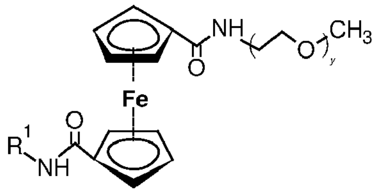 (IIe); 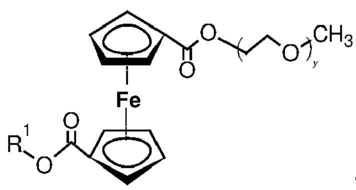 (IIf) ;

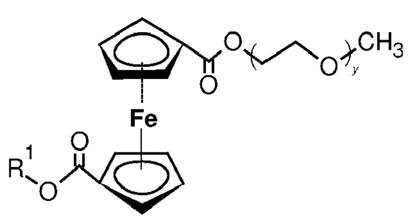 (IIg) 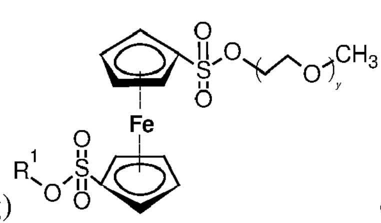 (IIh) ;

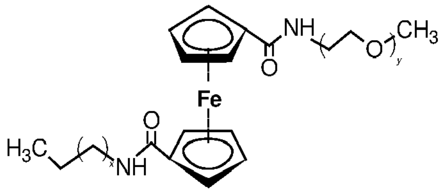
(IIi);

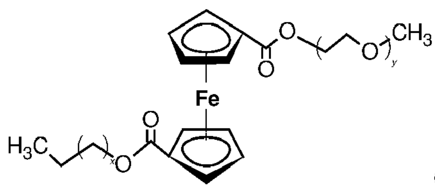
(IIk) ;

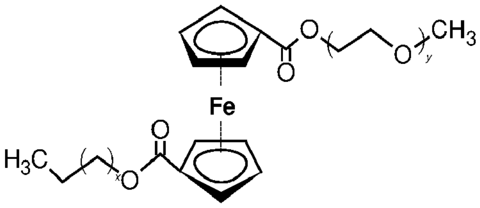
(III)

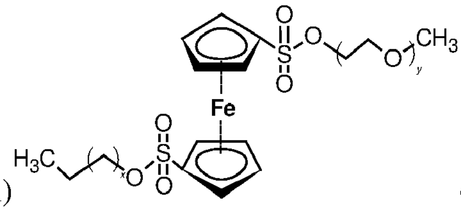
(IIm) ;

wherein $R^1$ is a **hydrophobic moiety** consisting of a substituted or unsubstituted, saturated or unsaturated, aliphatic $C_4$-$C_{36}$ chain-containing moiety;
wherein $R^2$ is a **hydrophilic moiety;**
wherein x is equal or superior to 2, in particular ranges from 2 to 34 ;
wherein y ranges from 8 to 100.

According to some of the said particular embodiments, the ferrocenyl-derivative compounds of the invention are of formula (IIa) or (IIb) or (IIc) or (IId) or (IIe) or (IIf) or (IIg) or (IIh) or (IIi) or (IIj) or (IIa) or (IIk) or (III) or (IIm), and are further characterized in that y ranges from 30 to 60.

According to some of the said particular embodiments, the ferrocenyl-derivative compounds of the invention are of formula (IIa) or (IIb) or (IIc) or (IId) or (IIe) or (IIf) or (IIg) or (IIh) or (IIi) or (IIj) or (IIa) or (IIk) or (III) or (IIm), and are further characterized in that: x is equal or superior to 2, in particular equal or superior to 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33 or 34.

According to a **second main embodiment,** the invention relates to a biocompatible micelle composition, characterized in that the micelle comprises one or more ferrocenyl-derivative compound(s) according to the invention, or a pharmaceutically acceptable salt thereof.

A ferrocenyl-derivative compound(s) according to the invention, or a pharmaceutically acceptable salt thereof may be selected from any of the ferrocenyl-derivative compound(s) described herein; and in particular any of the ferrocenyl-derivative compounds of the invention of formula (I) or (I') or or (Ia) or (Ib) or (Ic) or (Id) or (Ie) or (If) or (Ig) or (Ih) or (IIa) or (IIb) or (IIc) or (IId) or (IIe) or (IIf) or (IIg) or (IIh) or (IIi) or (IIj) or (IIa) or (IIk) or (III) or (IIm).

According to an exemplified embodiment, the ferrocenyl-derivative compound(s) are of formula (IIi):

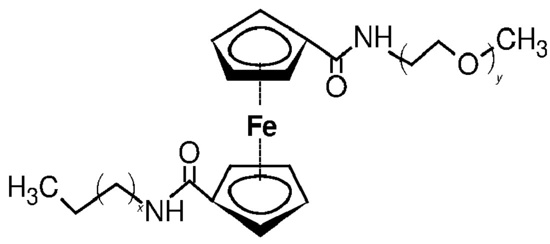
;

or a pharmaceutically acceptable salt thereof ;
wherein x is equal or superior to 2, in particular ranges from 2 to 34 ;
wherein y ranges from 8 to 100, in particular ranges from 30 to 60.

According to a **second main embodiment,** the invention relates to a biocompatible micelle composition, characterized in that the micelle comprises one or more ferrocenyl-derivative compound(s) according to the invention, or a pharmaceutically acceptable salt thereof.

According to some particular embodiments, the invention relates to a biocompatible micelle composition, characterized in that the micelle **comprises** one or more ferrocenyl-derivative compound(s), or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentadienyl ring is substituted with at least one **hydrophilic moiety,** said hydrophilic moiety being different from -OH, -C(=O)H or - C(=O)OH.

According to some particular embodiments, the invention relates to a biocompatible micelle composition, characterized in that the micelle **comprises** one or more ferrocenyl-derivative compound(s), or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentadienyl ring is substituted with at least one **hydrophilic moiety,** said hydrophilic moiety being different from -OH, or -$C_x$(=O)H or - $C_x$(=O)OH, with x being equal to 1, 2 or 3.

According to some embodiments, the invention relates to a biocompatible micelle composition, characterized in that the micelle **consists** of one or more of the said ferrocenyl-derivative compound(s), or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentandienyl ring is substituted with at least one **hydrophilic moiety,** said hydrophilic moiety being different from -OH, -C(=O)H or - C(=O)OH.

According to some particular embodiments, the invention relates to a biocompatible micelle composition, characterized in that the micelle **consists** of one or more of the said ferrocenyl-derivative compound(s), or a pharmaceutically acceptable salt thereof, characterized in that:

- the ferrocenyl moiety comprises two substituted cyclopentadienyl rings;
- one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
- the other cyclopentandienyl ring is substituted with at least one **hydrophilic moiety,** said hydrophilic moiety being different from -OH, - or -$C_x$(=O)H or - $C_x$(=O)OH, with x being equal to 1, 2 or 3.

According to some particular embodiments, the biocompatible micelle composition according to the invention is characterized in that it has an average hydrodynamic diameter equal or inferior to 40 nm; for example an average hydrodynamic diameter equal or inferior to 40, 39, 38, 37, 36, 35, 34, 33, 32, 31, 30, 29, 28, 27, 26, 25, 24, 23, 22, 21, 20, 19, 18, 17, 16 or 15 nm.

According to some particular embodiments, the biocompatible micelle composition according to the invention is characterized in that the micelle comprises at least one compound and/or polypeptide and/or nucleic acid; in particular at least one hydrophobic compound.

According to some particular embodiments, the biocompatible micelle composition according to the invention is characterized in that the micelle encapsulates at least one compound and/or polypeptide and/or nucleic acid; in particular at least one hydrophobic compound.

According to some particular embodiments, the biocompatible micelle composition according to the invention is characterized in that the micelle comprises at least one long wavelength absorbing photosensitizer, the said photosensitizer having an absorption band equal or superior to 620 nm, in particular ranging from 620 nm to 1200 nm; most preferably selected from a group consisting of: phenothiazine, porphyrins, chlorins, bacteriochlorins, cyanine, phtalocyanines, and derivatives thereof.

According to some particular embodiments, the biocompatible micelle composition according to the invention is characterized in that the micelle encapsulates at least one long wavelength absorbing photosensitizer, the said photosensitizer having an absorption band equal or superior to 620 nm, in particular ranging from 620 nm to 1200 nm; most

preferably selected from a group consisting of: phenothiazine, porphyrins, chlorins, bacteriochlorins, cyanine, phtalocyanines, and derivatives thereof.

According to some even more particular embodiments, the said photosensitizer is characterized by an absorption band ranging at least from 620 nm to 800 nm, and/or from 1000 nm to 1200 nm.

According to exemplary embodiments, a long wavelength abosrbing photosensitizer may be selected from the group of bacteriochlorins, and derivatives thereof, and preferably tetraphenylbacteriochlorin **(TPBC).**

The biocompatible micelle compositions or ferrocenyl-derivative compounds according to the invention are particularly considered for use as a medicament.

The biocompatible micelle compositions or ferrocenyl-derivative compounds according to the invention are particularly considered for use in a method of diagnosis.

The biocompatible micelle compositions or ferrocenyl-derivative compounds according to the invention are particularly considered for use for inducing ferroptosis in a subject, cell or cell sample.

Advantageously, the biocompatible micelle compositions or ferrocenyl-derivative compounds according to the invention are particularly considered for use in a method for treating or preventing a proliferative disorder.

More particularly, the biocompatible micelle compositions or ferrocenyl-derivative compounds according to the invention are particularly considered for use in a method for treating or preventing a proliferative disorder selected from the group consisting of: triple-negative breast cancer (TNBC), clear-cell RCC (ccRCC), non-neuroendocrine small cell lung cancer (SCLC), drug-resistant myeloproliferative disorders, metastatic myeloproliferative disorders.

According to particular embodiments, the biocompatible micelle compositions or ferrocenyl-derivative compounds according to the invention are for use in radiotherapy, photodynamic therapy, photodiagnosis or photodynamic inactivation.

According to a **third main embodiment,** the invention relates to a pharmaceutical composition comprising a biocompatible micelle according to the invention, or a ferrocenyl-derivative compound, or pharmaceutically acceptable salt thereof, according to the invention.

The pharmaceutical composition may be characterized in that it comprises:

- the biocompatible micelle, ferrocenyl-derivative compound, or pharmaceutically acceptable salt thereof, according to the invention;
- a pharmaceutically acceptable carrier.

According to a **fourth main embodiment,** the invention relates to a kit comprising:

- one or more ferrocenyl-derivative compound(s), or pharmaceutically acceptable salt thereof, according to the invention, or a biocompatible micelle composition according to the invention;
- one or more photosensitizer(s) having an absorption band equal or superior to 620 nm, in particular ranging from 620 nm to 1200 nm; most preferably selected from a group consisting of: phenothiazine, porphyrins, chlorins, bacteriochlorins, cyanine, phtalocyanines, and derivatives thereof.

According to a **fifth main embodiment,** the invention relates to a method for preparing a ferrocenyl-derivative compound, or pharmaceutically acceptable salt, according to the invention, comprising the steps of:

a) providing a precursor ferrocenyl-derivative compound comprising two cyclopentadienyl rings, each of the said rings being substituted with a reactive group;
b) bringing the precursor ferrocenyl compound derivative into contact with (i) a **hydrophobic moiety** consisting of an aliphatic chain-containing moiety, the aliphatic chain having at least 4 carbon atoms, and (ii) a **hydrophilic moiety,** said hydrophilic moiety being different from -OH, -C(=O)H or - C(=O)OH;
c) recovering the ferrocenyl-derivative compound.

In particular, the invention relates to a method for preparing a ferrocenyl-derivative compound, or pharmaceutically acceptable salt, as defined above, wherein step b) comprises:

b1) bringing the precursor ferrocenyl-derivative compound derivative into contact with (i) a **hydrophobic moiety** consisting of an aliphatic chain-containing moiety, the aliphatic chain having at least 4 carbon atoms;
b2) bringing the compound of step b1) into contact with a hydrophilic moiety, said hydrophilic moiety being different from -OH, -C(=O)H or - C(=O)OH;
steps b1) and b2) being preferably achieved in the presence of triethylamine and chloroform.

According to a **sixth main embodiment,** the invention relates to a method for preparing a biocompatible micelle

composition according to the invention, comprising a step of:

a) providing a composition comprising ferrocenyl-derivative compounds, or pharmaceutically acceptable salts thereof, according to the invention, and optionally a wavelength absorbing photosensitizer having an absorption band equal or superior to 620 nm;
b) ultra-sonicating the composition of step a);

thereby preparing the micelle composition.

Herein are further disclosed therapeutic methods comprising a step of administering the ferrocenyl-derivative compounds or pharmaceutically acceptable salts thereof, according to the invention, and/or biocompatible micelle according to the invention, to an individual/patient in need thereof.

Herein are further disclosed radiotherapy and/or photodynamic therapy methods comprising a step of administering the ferrocenyl-derivative compounds or pharmaceutically acceptable salts thereof, according to the invention, and/or a biocompatible micelle according to the invention, to an individual/patient/subject in need thereof.

More particularly, the biocompatible micelle may comprise and/or encapsulates a compound, polypeptide or nucleic acid; preferably, the biocompatible micelle comprises, or encapsulates, a wavelength absorbing photosensitizer such as a wavelength absorbing photosensitizer having an absorption band equal or superior to 620 nm.

Advantageously, said methods may comprise a step of bringing into contact the subject with an ionizing radiation and/or a light source, in particular bringing into contact a tumor tissue of the subject with an ionizing radiation.

Thus according to a particular embodiment, the present disclosure relates to a radiotherapy method in a subject in need thereof, comprising a step of:

a) administering a biocompatible micelle according to the disclosure;
b) bringing into contact the subject with a ionizing radiation, in particular bringing into contact a tumor tissue of the subject with a ionizing radiation.

According to a particular embodiment, the present disclosure relates to a photodynamic therapy in a subject in need thereof, comprising a step of:

a) administering a biocompatible micelle composition according to the disclosure, said micelle comprising a wavelength absorbing photosensitizer according to the disclosure, preferably having an absorption band equal or superior to 620 nm ;
b) bringing into contact the subject with a light source, in particular bringing into contact a tumor tissue of the subject with a light source at the absorption band of the photosensitizer.

According to a particular embodiment, the present disclosure relates to a radiotherapy or photodynamic therapy method in a subject in need thereof, comprising a step of bringing into contact the subject with a ionizing radiation or a light source ; characterized in that the subject in need thereof has been administered a biocompatible micelle composition according to the disclosure.

Herein are further disclosed *in vitro* or *ex vivo* methods for inducing ferroptosis or cell death in a cell sample, comprising a step of bringing into contact the cell with a biocompatible micelle comprising a wavelength absorbing photosensitizer according to the disclosure, preferably having an absorption band equal or superior to 620 nm, and then bringing into contact the cell with a light source at the absorption band of the photosensitizer.

Herein are further disclosed *in vitro* or *ex vivo* methods for introducing a compound a compound in a cell sample, comprising a step of bringing into contact the cell with a biocompatible micelle comprising (i) a wavelength absorbing photosensitizer according to the disclosure, preferably having an absorption band equal or superior to 620 nm and (ii) a compound to be introduced ; and then bringing into contact the cell sample with a light source at the absorption band of the photosensitizer.

A cell sample as described herein may, in particular, comprise eukaryotic cells and/or cells derived from a tumor tissue.

## General definitions

As used in this specification and the appended claims, the singular forms **"a", "an"** and **"the"** include plural referents unless the content clearly dictates otherwise.

As used in this specification and the appended claims, the term **"at least one",** may thus include one, or **"more than one".** Accordingly, the terms **"a plurality of"** or **"more than one"** may thus include « **two** » or « **two or more** ».

The term **"comprise"** is to be interpreted as specifying the presence of the stated features, integers, steps or

components, but not precluding the presence of one or more other features, integers, steps or components, or group thereof. Also, it may specify strictly the stated features, integers, steps or components, and therefore in such case it may be replaced with **"consist of"**.

As used herein, the term « **micelle** » refers to the aggregate of amphiphilic molecules or « **surfactants** », dispersed in a liquid. Micelles can thus be defined as colloidal dispersions from amphiphilic molecules with a hydrophobic tail and a hydrophilic head. Such micelles form in the liquid after reaching the corresponding critical micelle concentration (or CMC) of the amphiphile/surfactant(s). Hence, the term may encompass both micelles assembled from one type of amphiphile and micelles assembled from a plurality of distinct amphiphiles; such as those micelles assembled from two or more distinct amphiphiles. Micelles may be characterized from other types of aggregates by their organisation into one or more ordered layers, and in particular monolayers. Unless specified otherwise, the term may encompass micelles having all ranges of hydrodynamic size, in particular those having an hydrodynamic diameter at or below 40 nm, such as at or below 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8 or 7 nm.

As used herein, the term « **amphiphile** » refers to molecules and/or components (e.g., functional groups/moieties,polymers, and blocks of block polymers, etc.) of molecules having at least one hydrophilic moiety and at least one hydrophobic moiety, said hydrophilic and hydrophobic moieties being optionally linked by one or more linker regions. In particular, the term may refer to such molecules and/or components of such molecules, which are able to associate or co-associate in order to form micelles at or above a given CMC.

As used herein, the term **"hydrophilic"** refers to molecules and/or components (e.g., functional groups/moieties, polymers, and blocks of block polymers, etc.) of molecules having at least one hydrophilic group, and the term **"hydrophobic"** refers to molecules and/or components (e.g., functional groups/moieties, polymers, and blocks of block copolymers etc.) of molecules having at least one hydrophobic group.

Hydrophilic molecules or components thereof tend to have ionic and/or polar groups, and hydrophobic molecules or components thereof tend to have nonionic and/or nonpolar groups. Hydrophilic molecules or components thereof tend to participate in stabilizing interactions with an aqueous solution, including hydrogen bonding and dipole-dipole interactions. Examples of hydrophilic molecules may comprise PEG polymers.

Hydrophobic molecules or components tend not to participate in stabilizing interactions with an aqueous solution and, thus often cluster together in an aqueous solution to achieve a more stable thermodynamic state.

As used herein, the terms **"average droplet size"** or **"average hydrodynamic size"** or **"average hydrodynamic diameter"** are used interchangeably. The average droplet size of a particle or droplet or micelle can be determined by any dynamic light scattering (DLS) technique, for example a dynamic light scattering measurement by a Cordouan Vasco-Flex apparaturs, equipped with a 450 nm laser, at ambient temperature (e.g. at 20°C or 25°C).

As used herein, the term **"semi-fluorinated"** refers to chemical compounds having at least one fluorine atom, for example molecules having at least one carbon - fluorine bond.

As used herein, the term **"fluorocarbons"** refer to chemical compounds that contain at least one carbon-fluorine bond.

As used herein, the term **"perfluorinated"** and **"perfluorocarbon"** refers to chemical compounds that are analogs of hydrocarbons wherein all hydrogen atoms in the hydrocarbon are replaced with fluorine atoms. Perfluorinated molecules can also contain a number of other atoms, including bromine, chlorine, and oxygen. A bromine substituted perfluorocarbon is a perfluorocarbon wherein one or more of the fluorine atoms have been replaced with a bromine atom. A chlorine substituted perfluorocarbon is a perfluorocarbon wherein one or more of the fluorine atoms have been replaced with a chlorine atom. A chlorine and bromine substituted perfluorocarbon is a perfluorocarbon wherein one or more of the fluorine atoms have been replaced with a chlorine atom and wherein one or more of the fluorine atoms have been replaced with a bromine atom. Examples of perfluorocarbons which may be suitable as a hydrophobic perfluorocarbonated moiety include those selected from perfluoroalkanes, perfluoroalkylamines, perfluoro-crown-ethers, perfluorinated alcohols, perfluorohaloalkanes, perfluorinated carboxylic acids, perfluorinated azides, perfluorinated thiols, perfluorinated alkenes, perfluorinated alkynes, perfluorinated acrylates, and perfluorinated esters.

Examples of hydrophobic perfluorinated moieties/groups may be selected from the group consisting of: perfluorinated substituted $C_1$-$C_{30}$ alkyl groups, perfluorinated unsubstituted $C_1$-$C_{30}$ alkyl groups, perfluorinated substituted alkoxy groups, perfluorinated unsubstituted alkoxy groups.

As used herein, the term **"group"** may refer to a functional group of a chemical compound. For the purpose of the present disclosure, the terms **"group"** and **"moiety"** may be used interchangeably. Groups of the present compounds refer to an atom or a collection of atoms that are a part of the compound. Groups of the present invention may be attached to other atoms of the compound via one or more covalent bonds. Groups may also be characterized with respect to their valence state. The present term may thus also include groups characterized as monovalent, divalent, trivalent, etc. valence states.

As used herein, the term **"substituted"** refers to a compound wherein a hydrogen is replaced by another functional group.

As used herein, the term **"polymer"** refers to a molecule comprising a plurality of repeating chemical groups, typically referred to as monomers.

As used herein, the term **"copolymer",** also commonly referred to as a heteropolymer, is a polymer formed when two or more different types of monomers are linked in the same polymer.

As used herein, the term **"block copolymer"** refers to a type of copolymer comprising blocks or spatially segregated domains, wherein different domains comprise different polymerized monomers. In a block copolymer, adjacent blocks are constitutionally different, i.e. adjacent blocks comprise constitutional units derived from different species of monomer or from the same species of monomer but with a different composition or sequence distribution of constitutional units. Different blocks (or domains) of a block copolymer may reside on different ends of a polymer (e.g. [A] [B]), or may be provided in a selected sequence ([A][B][A][B]).

As used herein, the term **"diblock copolymer"** refers to block copolymers having two different chemical blocks. **"triblock copolymer"** refers to block copolymers having three different chemical blocks.

Block copolymers may include block copolymers having a first block comprising a first polymer such as a PEG polymer, for example a PEG polymer having 8 to 300 monomers, a second polymer, an intermediate block such as a fluorocarbon, including but not limited to, a fluorocarbon such as a fluorinated or perfluorinated alkane, and a third interior hydrophobic block. Block copolymers of the present invention are capable of undergoing self-assembly to make supramolecular structures, such as encapsulated droplets.

As used herein, the term "block copolymer" may thus include compounds comprising at least a first block, said first block optionally comprising or consisting of a first polymer such as PEG polymer, and at least a second block, said second block optionally comprising or consisting of a second polymer. The term « block copolymer » may also include functionalized block copolymers, such as copolymers having additional moieties.

As used herein, the terms **"pharmaceutically acceptable salt"** or **"physiologically acceptable salt"** may refer to salts which are formed from acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, palmoic acid, alginic acid, polyglutamic acid, naphthalene sulfonic acid, naphthalene disulfonic acid, and polygalacturonic acid. Suitable physiologically acceptable acid addition salts of compounds, for example of amphiphilic molecules, include hydrobromide, tartrate, citrate, trifluoroacetate, ascorbate, hydrochloride, tosylate, triflate, maleate, mesylate, formate, acetate and fumarate.

As used herein, the term **"halogen"** may, in particular, refer to chlorine, fluorine, bromine, or iodine.

As used herein, the term **"alkyl"** may refer to linear or branched alkyl groups. In general alkyl groups include those having from 1 to 30 carbon atoms; which may thus include those having from 1 to 2, 3, 4, 5, 6, 7, 8, 9, 10, 11, 12, 13, 14, 15, 16, 17, 18, 19, 20, 21, 22, 23, 24, 25, 26, 27, 28, 29 and 30 carbon atoms. For example $C_1$ to $C_x$ alkyl groups, also referred as ($C_1$-$C_x$) alkyls, may include ($C_1$-$C_2$) alkyls, ($C_1$-$C_3$) alkyls, ($C_1$-$C_4$) alkyls, (Ci-Cs) alkyls and ($C_1$-$C_6$) alkyls. Examples are, but are not limited to, methyl, ethyl, 1-propyl, 2-propyl, butyl, pentyl.

As used herein, the term **"substituted alkyl"** may include, among other alkyls, fully halogenated (e.g. perfluorinated) or semihalogenated alkyl groups, such as alkyl groups having one or more hydrogens replaced with one or more fluorine atoms, chlorine atoms, bromine atoms and/or iodine atoms. Substituted alkyl groups include fully fluorinated (i.e. perfluorinated) or semifluorinated alkyl groups, such as alkyl groups having one or more hydrogens replaced with one or more fluorine atoms. The term may also include, among other alkyls, those which are substituted with aryl groups, which in turn can be optionally substituted. Specific alkyl groups include methyl, ethyl, n-propyl, iso-propyl, cyclopropyl, n-butyl, s-butyl, t-butyl, cyclobutyl, n-pentyl, branched-pentyl, cyclopentyl, n-hexyl, branched hexyl, and cyclohexyl groups, all of which are optionally substituted.

As used herein, the term **"alkoxy"** may refer to an alkyl group (R) that has been modified by linkage to oxygen and can be represented by the formula (R-O) and can also be referred to as an alkyl ether group. Examples of alkoxy groups include, but are not limited to, methoxy, ethoxy, propoxy, butoxy and heptoxy. Alkoxy groups include substituted alkoxy groups wherein the alkyl portion of the groups is substituted as provided herein in connection with the description of alkyl groups.

As used herein the term **"carbonyl"** may refer to -(CO)-, wherein (CO) indicates that the oxygen is connected to the carbon with a double bond.

As used herein, the terms **"treat", "treating"** and **"treatment"** are meant to include alleviating or abrogating a disorder, disease, or condition, or one or more of the symptoms associated with the disorder, disease, or condition; or alleviating or eradicating the cause(s) of the disorder, disease, or condition itself.

As used herein, the terms **"prevent", "preventing",** and **"prevention"** mean reducing the risk of onset or slowing the occurrence of a given phenomenon, namely in the present invention, a cancer and/or dysplasia and more particularly, a pre-cancerous condition, an early stage cancer or a non-metastatic cancer. The term **"preventing"** also encompasses **"reducing the likelihood of occurrence"** or **"reducing the likelihood of reoccurrence"**.

As used herein, the term **"subject"** refers to an animal, including, but not limited to a human and non-human

mammal, including a primate (e.g., human), cow, sheep, goat, horse, dog, cat, rabbit, rat, or mouse. The terms "subject" and "patient" are used interchangeably herein in reference, in particular, to a mammalian subject, such as a human.

The terms **"tumor", "neoplasm", "proliferative disorder or disease"** and **"neoplastic disorder or disease"** are used interchangeably herein and are meant to refer to unwanted cell proliferation of one or more subset of cells in a multicellular organism resulting in harm (i.e., discomfort or decreased life expectancy) to the multicellular organisms. In certain embodiments, a tumor can be benign (non-invasive) or malignant (invasive).

As used herein, the term **"cancer"** is meant to refer to a malignant neoplasm, which is characterized by uncontrolled cell proliferation where cells have lost their normal regulatory controls that would otherwise govern the rate of cell growth. These unregulated, dividing cells can spread throughout the body and invade normal tissues in a process referred to as "metastasis". In the absence of other indications, the term is not construed to apply solely to one type of cancer, and may thus encompass those selected from: colorectal cancer, pancreatic cancer, lung cancer including non-small cell lung cancer, breast cancer, bladder cancer, gall bladder cancer, thyroid cancer, melanoma, liver cancer, uterine/cervical cancer, oesophageal cancer, kidney cancer, ovarian cancer, prostate cancer, head and neck cancer, and stomach cancer.

As used herein, the term **"contacting"** or **"contact"** is meant to refer to bringing together of a therapeutic agent and cell or tissue such that a physiological and/or chemical effect takes place as a result of such contact. Contacting can take place in vitro, ex vivo, or in vivo. In one embodiment, a therapeutic agent is contacted with a cell in cell culture (in vitro) to determine the effect of the therapeutic agent on the cell. In another embodiment, the contacting of a therapeutic agent with a cell or tissue includes the administration of a therapeutic agent to a subject having the cell or tissue to be contacted.

As used herein, the term **"photodynamic therapy"** or **"PDT"** refers to the act of bringing into contact a cell or tissue, especially a tumor cell or tumor tissue, with a given compound or composition, which is then activated as a "photosensitizer" by light (e.g. a laser or any other source of light such as LEDS) at a certain wavelength, in order to produce reactive oxygen species and kill said cell or tissue. Hence, PDT can be distinguished as an alternative to radiotherapy, although both strategies can be complementary.

## EXAMPLES

### Materials & Methods

#### *General*

Unless otherwise specified, all chemicals were purchased from Sigma-Aldrich and used without further purification. α-Methoxy- α -amino poly(ethylene)oxide 2000 was purchased from Iris Biotech. Flash chromatography was carried out on Kieselgel 60 (230-240 mesh, Merck). NMR spectra were recorded using a Bruker Avance DPX 400 spectrometer at 400 and 100 MHz respectively. Chemical shifts (*δ*) are given in ppm relative to the NMR solvent residual peak.

#### *Synthesis of ferrocene-derived compounds*

**$Fc(NHS)_2$ (1).** To a suspension of 1,1'-ferrocenedicarboxylic acid (100 mg, 0.36 mmol, 1.0 equiv.) in acetonitrile (5 mL) was added triethylamine (200 μL, 4.0 equiv.). The solution was stirred for 5 min at room temperature after which *N,N'*-disuccinimidyl carbonate (200 mg, 0.78 mmol, 2.2 equiv.) was added in one portion. After 3 h of stirring, water was added and the mixture was further stirred for 5 min. The orange precipitate was filtered and washed several times with water and then n-hexane. The resulting solid was dried in the oven at 100 °C for 1 h to afford an orange solid **(1,** 101 mg, 60%).

**$^1$H NMR** (400 MHz, DMSO-$d_6$) *δ:* 5.05 (m, 4H), 4.93 (m, 4H), 2.88 ppm (m, 8H).

**$^{13}$C NMR** (101 MHz, DMSO-$d_6$) *δ:* 170.5, 165.8, 75.7, 72.5, 66.2, 25.5 ppm.

**FT-IR** ($cm^{-1}$): 1761, 1726, 1072.

**C18-Fc-NHS (2).** To a solution of **1** (75 mg, 0.17 mmol) and triethylamine (50 μL, 0.37 mmol, 2.2 equiv.) in chloroform (20 mL) was added in one portion octadecylamine (39 mg, 0.14 mmol, 0.8 equiv.). The mixture was heated at 40 °C and stirred vigourously under argon atmosphere for 16 h. After cooling down to room temperature, the solvent was evaporated and the resulting crude product was purified on silica gel using cyclohexane/ethyl acetate (5:5 *v/v, $R_f$* = 0.41) to afford **2** as an orange-yellowish solid (59 mg, 56%)

**$^1$H NMR** (400 MHz, $CDCl_3$) *δ:* 6.60 (t, 1H), 4.88 (dd, 2H), 4.83 (dd, 2H), 4.67 (dd, 2H), 4.47 (dd, 2H), 2.93 (m, 4H), 1.52 (m, 2H), 1.40-1.20 (m, 32H), 0.88 ppm (t, 3H).

**FT-IR** ($cm^{-1}$): 2918, 2850, 1770, 1741, 1076.

**C18-Fc-PEG2k (3).** A solution of 2 (59 mg, 0.08 mmol), α-methoxy-ω-amino poly(ethylene)oxide 2000 (228

mg, 0.11 mmol, 1.2 equiv.) and triethylamine (50 μL, 0.37 mmol, 4.0 equiv.) in $HCCl_3$ (5 mL) was stirred at room temperature for 15 h. The solvent was evaporated and the crude product was purified on silica gel using $CH_2Cl_2$/MeOH (9:1, v/v then 8:2, $R_f$ = 0.35) to afford **3** as a yellow solid (131 mg, 65%).

**$^1$H NMR** (400 MHz, $CDCl_3$) δ: 7.10 (t, 1H), 7.03 (t, 1H), 4.57 (dd, 2H), 4.52 (dd, 2H), 4.36 (dd, 2H), 4.35 (dd, 2H), 3.81-3.53 (m, 178H), 3.41-3.33 (m, 2H), 3.37 (s, 3H), 1.64 (m, 2H), 1.42-1.20 (m, 32H), 0.87 ppm (t, 3H).

**$^{13}$C NMR** (101 MHz, $CDCl_3$) δ: 72.1, 71.3, 71.0, 70.8, 59.2, 40.1, 32.0, 30.0, 29.8, 22.9, 14.2 ppm.

**FT-IR** ($cm^{-1}$): 2883, 1101, 962, 841.

**C18-NHS (4).** A solution of stearic acid (1.0 g, 3.51 mmol), N-hydroxysuccinimide (606 mg, 5.27 mmol, 1.5 equiv.) and EDC.HCl (1.01 g, 5.27 mmol, 1.5 equiv.) in $CH_2Cl_2$/THF (5:15, v/v) was stirred under argon atmosphere for 18 h at room temperature. The solution was evaporated and extracted with $CH_2Cl_2$ and the combined organic layers were dried over $MgSO_4$, filtered and concentrated to afford product **4** as a white solid (1.045g, 78%).

**$^1$H NMR** (400 MHz, $CDCl_3$) δ: 2.83 (m, 4H), 2.60 (t, 2H), 1.74 (qt, 2H), 1.40 (m, 2H), 1.36-1.21 (m, 28H), 0.88 ppm (t, 3H).

**$^{13}$C NMR** (101 MHz, $CDCl_3$) δ: 169.3, 168.8, 32.1, 31.1, 29.8, 29.7, 29.5, 29.2, 28.9, 25.7, 24.7, 22.8 ppm.

**FT-IR** ($cm^{-1}$): 2918, 2848, 1785, 1724, 1070.

**C18-PEG2k.** A solution of **4** (50 mg, 0.13 mmol), α-methoxy-ω-amino poly(ethylene)oxide 2000 (383 mg, 0.19 mmol, 1.5 equiv.) and triethylamine (100 μL, 0.74 mmol, 6.0 equiv.) in $HCCl_3$ (3 mL) was stirred at room temperature for 17 h. The solution was evaporated and the crude product was purified on silica gel using $CH_2Cl_2$/MeOH (9: 1, v/v then 8:2, $R_f$ = 0.37) to afford 5 as a white solid (182 mg, 61%).

**$^1$H NMR** (400 MHz, $CDCl_3$) δ: 6.18 (t, 1H), 3.81-3.45 (m, 180H), 3.37 (s, 3H), 2.16 (t, 2H), 2.51 (qt, 2H), 1.34-1.20 (m, 30H), 0.87 ppm (t, 3H).

**$^{13}$C NMR** (101 MHz, $CDCl_3$) δ: 171.9, 71.4, 70.0, 69.6, 69.4, 58.5, 38.7, 36.1, 31.4, 29.2, 29.0, 28.9, 28.8, 24.9, 22.2, 13.6 ppm.

**FT-IR** ($cm^{-1}$): 2885, 1639, 1554, 1101.

***Synthesis of bacterochlorin (TPBC)***

A solution of tetra-phenylporphyrin **(TPP)** (1.16 mmol, 100 mg, 1.0 equiv.), anhydrous $Na_2CO_3$ (3.96 mmol, 420 mg, 24.0 equiv.) and tosyl hydrazide (1.63 mmol, 304 mg, 10.0 equiv.) in pyridine (7.5 mL) was stirred and heated at 100 °C for 12 h under argon. The mixture was allowed to cool down to room temperature. Benzene (100 mL) and water (50 mL) were added. The organic phase was washed in the following order: 3 N HCl, $H_3PO_4$, saturated $NaHCO_3$ and water. All washes were done 3 times per reagent and using 50 mL each time. The organic layer was dried on $MgSO_4$, filtered and evaporated. TPBC was obtained as a purple solid (66% yield). The UV-vis spectrum is in accordance with the literature, showing no presence of TPP or tetraphenylchlorin (TPC).

***Encapsulation of hydrophobic molecules***

**Preparation of NR@C18-Fc-PEG2k and NR@C18-PEG2k.** To a solution of **C18-Fc-PEG2k** or **C18-PEG2k** (10 mg $mL^{-1}$) in water (2 mL) was added a solution of Nile Red (NR, 400 μL, 1 mg $mL^{-1}$) in chloroform (1 mL). The mixture was sonicated using an ultrasound sonicator probe (power 40%, 10 min, pulse) allowing NR to be gradually encapsulated by evaporation of chloroform *via* heating of the probe. The resulting solution was filtered on 0.45 μm nylon membrane to eliminate any free NR from the medium.

**Preparation of DPBF@C18-PEG2k and TPBC@C18-PEG2k**. To a solution of **C18-Fc-PEG2k** (10 mg $mL^{-1}$) in water (1 mL) was added a solution of **TPBC** (bacteriochlorin) or **DPBF** (1,3-diphenylisobenzofuran) (12.5 μL, 1 mg $mL^{-1}$) in chloroform (1 mL). The mixture was sonicated using an ultrasound sonicator probe (power 40%, 10 min, pulse) allowing **TPBC** or **DPBF** to be gradually encapsulated by evaporation of chloroform *via* heating of the probe. The resulting **TPBC/DPBF@C18-Fc-PEG2k** colloidal suspension was filtered on 0.45 μm nylon membrane to eliminate any free **TPBC** or **DPBF** from the medium.

**Preparation of TPBC@C18-Fc-PEG2k.** To a solution of **C18-Fc-PEG2k** (10 mg $mL^{-1}$) in water (1 mL) was added a solution of **TPBC** (25 μL, 1 mg $mL^{-1}$) in chloroform (1 mL). The mixture was sonicated using an ultrasound sonicator probe (power 40%, 10 min, pulse) allowing **TPBC** to be gradually encapsulated by evaporation of chloroform *via* heating of the probe. The resulting **TPBC@C18-Fc-PEG2k** colloidal suspension was filtered on 0.45 μm nylon membrane to eliminate any free **TPBC** from the medium.

**Preparation of TPBC-DPBF@C18-PEG2k.** To a solution of **C18-Fc-PEG2k** (10 mg $mL^{-1}$) in water (1 mL) was added a solution of **TPBC** (12.5 μL, 1 mg $mL^{-1}$) in chloroform (1 mL). The mixture was sonicated using an ultrasound sonicator probe (power 40%, 10 min, pulse) allowing **TPBC** to be gradually encapsulated by evaporation of chloroform *via* heating of the probe. The resulting **TPBC@C18-Fc-PEG2k** colloidal suspension was filtered on 0.45 μm nylon membrane to eliminate any free **TPBC** from the medium. To this suspension was then added a solution of **DPBF** (50

μL, 1 mg $mL^{-1}$) in chloroform (1 mL). The resulting mixture was sonicated in the same conditions as previously. After filtration on a 0.45 μm nylon membrane, **TPBC-DPBF@C18-PEG2k** micelles were obtained.

***Determination of the Critical Micellar Concentration (CMC) by surface tension measurement***

Different dispersions of amphiphiles were prepared at variable concentrations: 0.001, 0.005, 0.01, 0.05, 0.1, 0.2, and 0.1 mg $mL^{-1}$. Surface tension was measured (using Kibron AquaPi Plus) in triplicate for each concentration, which gave two straight lines. The intersection of both lines corresponds to the point at which amphiphiles begin to self-assemble into micelles. From this concentration and higher, any added amphiphiles will form micelles.

***Micelles size analysis by Dynamic Light Scattering (DLS)***

Hydrodynamic diameters $D_h$ were recorded using Malvern Zetasizer Nano ZS at 25 °C (He-Ne laser 633 nm). Samples were prepared at the given concentration by solubilizing the solid amphiphiles in milliQ water (1 mL). Sonication by ultrasound bath or ultrasound probe and then filtration on 0.20 μm nylon membrane afforded a suspension of micelles that was used for DLS.

***Cytotoxicity assays***

Human breast cancer cell line (MCF-7) was used for all *in vitro* experiments. Cells were cultured in medium with dulbecco's modified eagle's medium (DMEM), fetal bovine serum (FBS) 10% (v/v), Pen-Strep 1% (v/v) and sodium pyruvate (1 mM). Before treatment with micelles, cells were washed with warm PBS, trypsinized and centrifuged at 200 ×g for 2 min. Supernatant was removed, cells were resuspended in fresh medium and filtered with a cell strainer 40 μM to remove cell aggregates. 100 μL of cell suspension were seeded with automation into the wells of 96-well plate (final: 1500 cells/well). 24 h after incubation, cells were treated with either **C18-PEG2k** or **C18-Fc-PEG2k** micelles. 50 μL of OptiMEM were layered on top of the culture wells (final micelle concentration ranging from 2 to 200 μM). Plates were incubated (37 °C, 5% $CO_2$) for 1 h. Supernatant was removed first partly by aspiration using Biotek ELX405. Remaining supernatant was removed by centrifuging the plate upside down on top of a paper towel (70 ×g, 10 sec). Then, 100 μL of fresh culture medium were immediatly added manually to the wells. When necessary, the plates were irradiated 10 min with the A160WE TB light at 8 cm from the bottom of the wells. Cells were then incubated (37 °C, 5% $CO_2$) for 72 h. Then, cells were fixed and stained by addition of 50 μL PFA (4% w/v final) and Hoechst 33342 (final: 2 μg $mL^{-1}$). Plates were incubated overnight at 4 °C, then supernatant was removed by aspiration and replaced by 100 μL PBS. Plates were aquired on the Operetta device.

***Irradiation experiments***

All samples were irradiated in a quartz cuvette (4 mL volume, 1 cm path length) and UV spectra were measured at various times depending on the experiment, using either the 460 (A160WE Tuna Blue) or 740 nm (PR160L) nm light source. Samples were irradiated with light source positioned 8 cm away with an irradiance of 55 mW $cm^{-2}$ for A160WE Tuna Blue. About PR160L-740, no data was available from the provider thus no irradiance could be provided. In case of A160WE Tuna Blue, the irradiance at 8 cm was calculated using experimental data from the provider at 5 cm with an irradiance of 140 mW $cm^{-2}$ and equation 1 (eq. 1), with $I_2$ = 140 mW $cm^{-2}$, $d_2$ = 5 cm and $d_1$ = 8 cm. For both lights, experiments were done at maximum power output (~40 W according to provider).

All light sources were purchased from Kessil (https://kessil.com/products/science main.php). Varian Cary® 50 UV-Vis spectrophotometer was used to perform all measurements.

$$\frac{I_1}{I_2} = \frac{d_2^2}{d_1^2} \qquad \text{(eq. 1)}$$

**Example 1. Synthesis of an amphiphile containing a ferrocene linker.**

For this study, two amphiphiles were synthesized, the first one, **C18-Fc-PEG2k (3),** containing the ferrocene linker, corresponding to the ferrocene derivative of **fig. 1,** and the second one being the control molecule without ferrocene **C18-PEG2k** (5). As shown on **Fig. 2,** intermediate **1** was obtained by activating the commercial 1,1'-ferrocenedicarboxylic acid using *N,N'*-disuccinimidyl carbonate in acetonitrile and triethylamine. Intermediate **1** was then reacted with an equimolar amount of octadecylamine in chloroform at 40 °C overnight to afford **2** with only 32% yield. This is explained by the fact that the reaction is not selective, leading to a mixture of mono- and di-substituted products, as well as unreacted

**1.** The desired amphiphile **3** was finally obtained by reacting **2** with α-methoxy-ω-amino poly(ethylene)oxide 2000 *(MW* = 2000) in dichloromethane at room temperature.

As for the control amphiphile without ferrocene, the two steps synthesis consisted of activating the commercially available stearic acid into the corresponding NHS-ester to afford **4.** The latter was then reacted with α-methoxy-ω-amino poly(ethylene)oxide 2000 to yield the desired amphiphile **C18-PEG2k (5).** Both amphiphiles **C18-Fc-PEG2k** and **C18-PEG2k** were successfully dispersed in water and were shown to self-assemble into micelles.

**Example 2. Micelle assembly and characterization**

Micelles were prepared by dispersing 3 in aqueous medium. The suspension was then sonicated using either an ultrasound bath or an ultrasound probe. Filtration on 0.45 μm membrane provided a colloidal suspension of micelles. The critical micelle concentration (CMC) was determined by tensiometry and found to be 0.048 mg $mL^{-1}$.

To further characterize the nanoobjects, a 10 mg $mL^{-1}$ suspension of **C18-Fc-PEG2k** in water, well above the CMC value, was analyzed by DLS. The DLS measurements revealed a hydrodynamic diameter ($D_h$) of 10 nm **(Fig. 3A).** This value is consistent with similar micelles bearing C18 and PEG2k chains. Hence, **C18-Fc-PEG2k** can efficiently and rapidly self-assemble into micelles in aqueous medium. Zeta potential was also measured as shown in **Fig. 3B,** resulting in charge surface near 0 mV. This was expected and coherent as nanoparticles coated with PEG have a neutral surface and the micelle colloids are sterically (not electrostatically) stabilized. The colloidal stability was monitored by DLS over 3 weeks and showed no aggregation or degradation of the micelles. The UV spectrum was acquired in water with the characteristic absorption band at 442 nm. This absorption band is ascribed to the charge transfer between the iron atom and the Cp rings, giving ferrocene its characteristic signature orange-yellowish color.

**Example 3. Photodegradation study ($\lambda_{irr}$ = 460 nm)**

Micelles obtained from 3 carry a photodegradable ferrocene moiety. Upon irradiation, ferrocene undergoes photolysis, releasing iron species and organic by-products. Carbonyl-substituted ferrocene derivatives are considered photolabile **(**Yamaguchi et al., Efficient Photodissociation of Anions from Benzoyl-Functionalized Ferrocene Complexes, Inorg. Chem. 1999, 38, 4861-4867). A 10 mg $mL^{-1}$ solution of **(3)** in water was irradiated using a 460 nm LED light source. After 5 min of irradiation, the clear orange solution became heterogeneous, with an apparent dark precipitate and a pale yellow supernatant (Fig. 5A). The supernatant and precipitate were separated by centrifugation and analyzed. The UV-vis spectrum of the supernatant showed almost no absorption band at 442 nm, which is assigned to the dissociation of the Fe-Cp bonds, proving the decomposition of the ferrocene unit (Fig.5B). Therefore, no ferrocene unit was detected by UV-vis spectroscopy in the supernatant after irradiation.

For the precipitate, we were expecting to generate ferric oxides or ferric hydroxides species during the photodegradation process. The dark precipitate was insoluble in any solvent, favoring the hypothesis of an inorganic product. We performed X-ray photoelectron spectroscopy (XPS) of amphiphile **C18-Fc-PEG2k** as synthesized in the solid form and compared the results to the analysis of the precipitate obtained after irradiation of the micelle colloid at 460 nm. The results obtained for the pristine sample displayed one peak at 708.3 eV and a satellite structure at 716.6 eV for the binding energy region of Fe-$2p_{3/2}$. These values are consistent with the literature describing $Fe^{2+}$ compounds, confirming that, prior to irradiation, the iron atom of the ferrocene unit has indeed an oxidation state of +2.

In the XPS analysis of the irradiation-induced precipitate, both the peak and satellite structure appeared shifted to higher binding energies, respectively at 711.0 and 719.6 eV. These values are characteristic of an $Fe^{3+}$ state, proving that oxidation occurred during the photodegradation, although a slight portion of $Fe^{2+}$ cannot be excluded. Apart from the shift to the higher binding energies, it is worth noting that the spectra was wider for the precipitate, a feature commonly observed in the presence of iron oxides or hydroxides species. Thus, we demonstrated that our micelles were photodegraded in water by oxidizing $Fe^{2+}$ from the ferrocene unit to $Fe^{3+}$, forming inorganic species.

To further prove the existence of $Fe^{3+}$ during the photoirradiation process, we conducted irradiation experiments using molecules that bind to $Fe^{2+}$ and $Fe^{3+}$. Potassium thiocyanate (KSCN), in presence of $Fe^{3+}$, leads to an intense blood-red color, whereas with $Fe^{2+}$, no significant color change is observed. $Fe^{2+}$ being unstable in solution in the presence of oxygen, a slight reddish color appears gradually due to oxidation of $Fe^{2+}$ to $Fe^{3+}$ over time. Potassium ferricyanide ($K_3[Fe(CN)_6]$) is also used in the detection of $Fe^{2+}$ and $Fe^{3+}$. Indeed, in presence of $Fe^{2+}$, a prussian blue precipitate is formed while, with $Fe^{3+}$, a clear green-to-brown solution appears. In our study, both complexing agents were used. **C18-Fc-PEG2k** micelles were irradiated with either KSCN or $K_3[Fe(CN)_6]$, and both gave positive results to $Fe^{3+}$ after irradiation. The usual dark precipitate from the photodegradation of the ferrocene unit was not observed with either KSCN or $K_3[Fe(CN)_6]$, showing that no oxide species were formed, most likely because complexation of the released $Fe^{3+}$ occurred instead of precipitation of the oxides.

To evaluate this hypothesis, from a newly irradiated **C18-Fc-PEG2k** sample, we separated the supernatant from the precipitate by centrifugation. Then, KSCN was added to the supernatant and the precipitate (resuspended in

water), and both led to negative tests, showing no color change. Hence, we concluded that i) no $Fe^{3+}$ ions were present in the supernatant and ii) once formed, ferric oxides/hydroxides species cannot be complexed by KSCN. This confirms the fact that KSCN is complexing $Fe^{3+}$ before the oxides/hydroxides can be formed during the irradiation process. The same observations were made with $K_3[Fe(CN)_6]$ instead of KSCN, confirming the photooxidation of $Fe^{2+}$ to $Fe^{3+}$.

The irradiation experiment was conducted in different solvents: *N,N'*-dimethylformamide (DMF), methanol, acetonitrile, chloroform and acetone with solutions of amphiphiles at 10 mg $mL^{-1}$. The objective was to determine whether or not the nature of the solvent could have a crucial role in the photodegradation process. In methanol, similar observations were made as in water, namely a dark precipitate and discoloration of the supernatant. In DMF, no such precipitate was detected but a very dark and cloudy solution was obtained, implying a photodegradation as well.

For the photodecomposition experiments in other aprotic solvents such as acetonitrile, chloroform and acetone, no macroscopic degradation was observed and the solutions remained orange-yellowish. To support this observation, UV measurements were performed. Following the irradiation in acetonitrile and acetone, a slight hypsochromic shift was observed while maintaining the same absorbance intensity, suggesting that no major structural modification to the ferrocene moiety had taken place. When irradiated in chloroform, not a single shift or decrease in absorbance was observed, both spectra before and after irradiation were identical. The slight alteration in acetone and acetonitrile can be explained by the fact that there might be traces of water in these solvents. Water seems to play a critical role in the photodegradation process. Chloroform is not miscible with water, hence the unmodified UV spectrum after irradiation. Moreover, we observed a very similar behavior in methanol, meaning that the protic nature and nucleophilicity of the solvent are probably the main reasons as to why the photodegradation occurs the fastest in water and methanol.

Without wishing to be bound by the theory, it is proposed that the nature of the solvent combined with acyl groups is responsible for the fast photodecomposition of the ferrocene unit using visible light. High electron density ferrocenes (pristine and donor substituents) tend to be less sensitive to photodecomposition due to strong metal-ligand bonds. However, substituting both Cp rings with acyl groups introduces strong metal-to-ligand charge transfer (MLCT). As a consequence, the hapticity of the Cp ring is reduced from $\eta^5$ to $\eta^4$, hence weakening the metal-ligand bond and thus allowing nucleophiles, such as solvent molecules, to attack and displace Cp rings by a heterolytic Fe-Cp cleavage process.

Knowing that micelles are photodegraded under 460 nm irradiation, we tried to monitor the photodegradation process as represented in **Fig. 6** by measuring the absorbance at 442 nm after different irradiation times, ranging from 0 to 15 min. After 30 seconds of irradiation, a 40% decrease of the 442 nm band was observed, and total disappearance was measured after 4 min. We irradiated up to 15 min and observed no evolution of the UV-vis profile. These results show that micelles according to the invention undergo fast photodegradation that can be exploited for the light-triggered induction of ferroptosis as well as simultaneous release of encapsulated hydrophobic molecules.

## Example 4. Encapsulation and release of a hydrophobic molecule

In order to evaluate the ability of micelles according to the present invention to encapsulate and release a potential toxic molecule, we decided to use Nile Red (NR) as a model of a hydrophobic compound.

Nile Red is a dye that has high extinction coefficient and is strongly fluorescent in lipophilic environment, but in water it precipitates and is quickly quenched. These properties are perfectly suited for the encapsulation of the dye into the hydrophobic core of our micelles. Upon irradiation of the micelles, their degradation is expected to induce the release and aggregation/precipitation of NR in the aqueous medium that can be monitored UV-vis spectroscopy.

NR (2 wt%, $\lambda_{abs}$ = 555 nm) was loaded into **C18-Fc-PEG2k** micelles following the standard procedure used in our laboratory (see Materials & Methods). After sonication and filtration over 0.45 $\mu$m membrane, the colloidal suspension of **NR@C18-Fc-PEG2k** was irradiated and the degradation was monitored by UV-vis spectroscopy **(Fig. 7A).**

In this case, exposure to light with $\lambda$= 460 nm triggered photodegradation of the micelles similar to what was observed with **C18-Fc-PEG2k** micelles. Indeed, after only 30 seconds of irradiation, around 40% of the NR signal was lost. As a consequence, the loss of NR could be correlated to the photodegradation of the ferrocene unit, leading to the disruption of the micelles and thus, the release of NR in the aqueous medium. The absorption band of NR totally disappeared after 2 to 4 min of irradiation, which is in accordance with the kinetics of ferrocene photodegradation.

To rule out the possibility of NR photobleaching during the irradiation process, the same experiment was carried out using micelles without ferrocene obtained from the self-assembly of **C18-PEG2k (Fig.** 7B). To this end, NR was encapsulated in **C18-PEG2k** micelles. The aqueous suspension of **NR@C18-PEG2k** was then exposed to 460 nm light for 15 min. The absorbance corresponding to NR remained the same, indicating that NR is stable under these conditions.

As a conclusion, we can doubtlessly state that the decrease of the absorbance at 555 nm is due to the release of NR from the micelles that are gradually degraded under 460 nm light, followed by NR precipitation.

## Example 5. Photodegradation study ($\lambda_{irr}$ = 740 nm) using TPBC

**C18-Fc-PEG2k** micelles are intended to treat solid tumors *in vivo.* However, the use of blue light is not optimal for such purpose as the penetration through biological tissue is limited. There are two wavelength windows of high tissue penetration, ranging from 620 to 800 nm (NIR-I region) and from 1000 to 1200 nm (NIR-II region), respectively.

In order to address this technical issue and improve the effect in treating solid tumors, we decided to encapsulate a photosensitizer (PS) in order to assist the photodegradation of **C18-Fc-PEG2k** micelles under NIR light irradiation.

Ferrocene only absorbs around 442 nm, therefore it cannot be photoactivated using a 740 nm lamp. However, iron(II) is very sensitive to oxidation and especially to reactive oxygen species (ROS). With this in mind, we decided to use tetraphenylbacteriochlorin **(TPBC)** as PS and encapsulate it within the micelles, as it can be photoactivated at 740 nm and generate singlet oxygen when irradiated. Bacteriochlorins are porphyrin derivatives in which two of the four pyrrolic rings are reduced.

Advantageously, **TPBC** shows minimal absorbance in the 400-500 nm region where ferrocene absorbs the most. It is thus possible to monitor both **TPBC** and ferrocene in the same sample without interference.

**TPBC** (0.25 wt%) was loaded into **C18-Fc-PEG2k** micelles following the procedure described for NR. To assess the photostability of **C18-Fc-PEG2k** at 740 nm, we conducted the same experiment as previously and observed no major change in the UV-vis profile **(Fig. 9A),** confirming that the micelles were stable under these conditions.

We then submitted **TPBC@C18-Fc-PEG2k** micelles to 740 nm light as depicted in **Fig. 9B.** After 4 min of irradiation, neither **TPBC** nor ferrocene could be detected by UV-vis spectroscopy anymore. Prolonging the irradiation over 15 min did not produce any noticeable change. Hence, coupling the 740 nm irradiation and **TPBC** enables a rapid and efficient degradation of the micelles. Indeed, the same degradation speed was observed under these new conditions compared to the use of 460 nm light without photosensitizer. Being able to degrade micelles according to the present disclosure with a near infrared lamp is thus very promising for *in vivo* biological applications.

## Example 6. Detection of singlet oxygen

In order to better understand the photodegradation using TPBC as photosensitizer, 1,3-diphenylisobenzofuran (DPBF) was used to confirm the putative generation of singlet oxygen during the irradiation process, and whether it is singlet oxygen production what mediates TPBC-assisted micelle photolysis with 740 nm light.

1,3-Diphenylisobenzofuran **(DPBF)** is a reagent for detecting singlet oxygen. Indeed, in presence of $^1O_2$, **DPBF** forms an unstable peroxide that decomposes into 1,2-dibenzoylbenzene **(DBB)** which is colorless. To assess its stability under NIR light, **DPBF** (solubilized in DMSO) was subjected to 740 nm light **(Fig. 8A)** and no alteration was observed after several minutes under irradiation. After assessing the stability of **DPBF** in DMSO, we added a solution of **TPBC** in DMSO and irradiated the mixture at 740 nm. The reaction was monitored using UV-vis spectroscopy **(Fig. 8B).**

Firstly, upon addition of **TPBC,** no modification of the **DPBF** maximum absorption band at 417 nm was observed. After 1 min of irradiation at 740 nm, a drastic decrease in absorbance was noticed, indicating that **DPBF** was degraded. This observation could be seen by naked eye, as the solution, initially green, turned colorless. However, as irradiating was extended over longer times, a decrease in **TPBC** absorption bands was observed. The resulting adduct 1,2-dibenzoylbenzene **(DBB),** from the decomposition of **DPBF** by singlet oxygen, was detected by mass spectrometry and characterized by $^1H$ NMR.

After proving the generation of singlet oxygen in solution, we tried to mimic the nanosystem used for the photodegradation of **TPBC@C18-Fc-PEG2k** by encapsulating **DPBF** and **TPBC** into ferrocene free micelles **C18-PEG2k** to provide **TPBC-DPBF@C18-PEG2k** micelles. As the degradation of **DPBF** was complete after only 60 sec of reaction in DMSO, we decided to follow the process every 20 sec up to 240 sec.

First, we ensured that both **TPBC** and **DPBF** could be encapsulated into our **C18-PEG2k** micelles individually. **Fig. 9A** shows that both hydrophobic molecules are not soluble in water (dashed lines), however, in presence of **C18-PEG2k** micelles, UV measurements indicate **DPBF** and **TPBC** characteristic absorption bands, proving their encapsulation into the micelles. Loading both molecules together turned out to be successful as both signals are clearly visible by UV, confirming that **TPBC-DPBF@C18-PEG2k** micelles were prepared. Since the medium changed, we had to assess **DPBF** stability under irradiation once again. As displayed on Fig. **9B,** DPBF is stable upon irradiation for 4 min. **TPBC-DPBF@C18-PEG2k** was then irradiated under 740 nm light and the photoreaction was monitored by UV-vis spectroscopy every 20 sec **(Fig. 9C).**

After only 20 sec of irradiation, **DPBF** completely disappeared by UV and naked eye. Further irradiation only showed photodegradation of **TPBC** as depicted on **Fig. 9D.** Indeed, the more **TPBC** was degraded, the more intense the absorption band at 420 nm was, corresponding to **TPP.**

As a conclusion, the degradation of **DPBF** using the photosensitizer coupled with a 740 nm light confirmed the generation of singlet oxygen during the photo-irradiation. The rapid degradation of **DPBF** loaded into the micelles highly suggests that **TPBC** degrades the ferrocene unit the same way *via* oxidation through the *in situ* generation of singlet

oxygen. As **TPBC** is hydrophobic, it is concentrated close to the degradable ferrocene moieties and this spatial proximity facilitates the whole process by micellar catalysis.

Accordingly, this is evidence that the micelle compositions according to the present invention are particularly convenient for the transport of hydrophobic compounds.

Example 7. Cytotoxicity assessment at $\lambda$**= 460 nm**

To study the cytotoxicity of our micelles, MCF-7 cells were incubated with **C18-Fc-PEG2k** or **C18-PEG2k** micelles from 200 μM to 2 μM **(Fig. 10).** Irradiation was applied for 10 min at 460 nm. 72 h after irradiation, cells treated with **C18-Fc-PEG2k** showed a dose-response effect, displaying a decrease of cell survivability for the highest concentration of micelles. Cells containing **C18-Fc-PEG2k** micelles that were not irradiated did not show any cytotoxicity.

In the meantime, MCF-7 cells were treated the same way using **C18-PEG2k** instead as a control group. In that case, no sign of cytotoxicity was observed neither with nor without irradiation.

As a consequence, we conlude that i) blue light is compatible with cells under our experimental conditions, ii) neither micelles with nor without ferrocene are harmful to the cells in absence of irradiation and iii) the ferrocene moiety is playing a key role in the observed cytotoxicity upon irradiation.

## Claims

1. Ferrocenyl-derivative compound, or a pharmaceutically acceptable salt thereof, **characterized in that**:

   - the ferrocenyl moiety comprises two substituted cyclopentadienyl rings;
   - one cyclopentadienyl ring is substituted with at least one **hydrophobic moiety** consisting of a substituted or unsubstituted, saturated or unsaturated, aliphatic chain-containing moiety, the aliphatic chain having at least 4 carbon atoms;
   - the other cyclopentandienyl ring is substituted with at least one **hydrophilic moiety,** said hydrophilic moiety being different from -OH, -C(=O)H or - C(=O)OH.

2. Ferrocenyl-derivative compound according to claim 1; wherein each cyclopentadienyl ring is selected from: a carbonyl (C=O)-substituted cyclopentadienyl ring, a nitro ($NO_2$)-substituted cyclopentadienyl ring, or a sulfonic acid ($SO_3H$)-substituted cyclopentadienyl ring.

3. Ferrocenyl-derivative compound according to claim 1 or 2, of formula (I) or (I'):

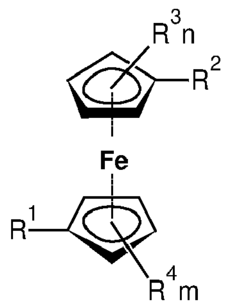
(I),
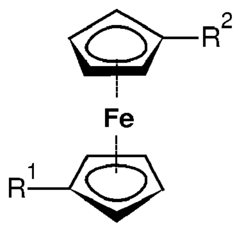
(I');

   wherein:

   n and m are identical or different, and equal to 0, 1, 2, 3 or 4;
   $R^1$ is a **hydrophobic moiety** consisting of a substituted or unsubstituted, saturated or unsaturated, aliphatic $C_4$-$C_{36}$ chain-containing moiety;
   $R^2$ is a **hydrophilic moiety,** said $R^2$ being different from -OH, -C(=O)H or - C(=O)OH;
   $R^3$ and $R^4$ are identical or different, and selected from H, or optionally substituted acyl, alkyl, alkenyl, aryl, cycloalkyl, alkaryl, aralkyl, heteroaryl and heterocycloalkyl groups; with the proviso that $R^3$ is not a substituted or unsubstituted, saturated or unsaturated, aliphatic $C_4$-$C_{36}$ chain-containing moiety.

4. Ferrocenyl-derivative compound according to claim 1 or 2, of formula (Ia) or (Ib) or (Ic) or (Id) or (Ie) or (If) or (Ig) or (Ih):

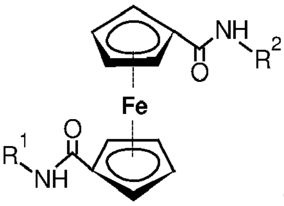 (Ia) ; 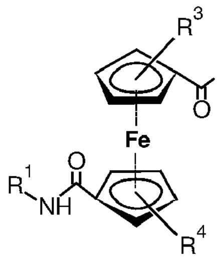 (Ib) ;

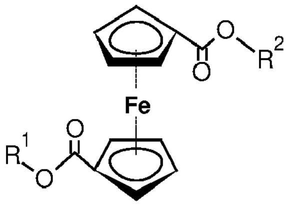 (Ic) ; 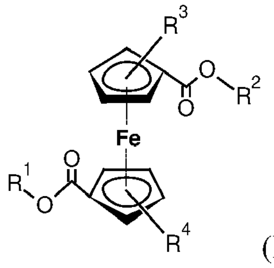 (Id) ;

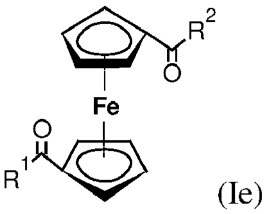 (Ie) ; 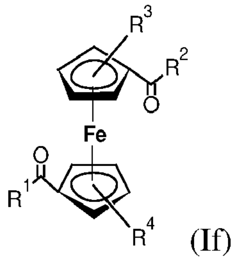 (If) ;

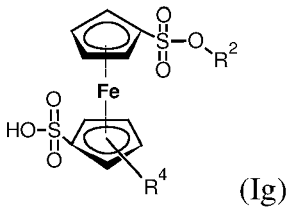 (Ig) ; 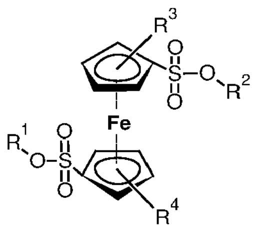 (Ih) ;

wherein $R^1$ is a **hydrophobic moiety** consisting of a substituted or unsubstituted, saturated or unsaturated, aliphatic $C_4$-$C_{36}$ chain-containing moiety;
$R^2$ is a **hydrophilic moiety;**
with the proviso that $R^2$ is not -H for formulae (Ic) and (Id);
with the proviso that $R^2$ is not -H nor -OH for formulae (Ie) and (If)
$R^3$ and $R^4$ are identical or different, and selected from H, or optionally substituted acyl, alkyl, alkenyl, aryl, cycloalkyl, alkaryl, aralkyl, heteroaryl and heterocycloalkyl groups; with the proviso that $R^3$ is not a substituted or unsubstituted, saturated or unsaturated, aliphatic $C_4$-$C_{36}$ chain-containing moiety.

**5.** Ferrocenyl-derivative compound according to any of the preceding claims; wherein the **hydrophilic moiety** is selected from a group consisting of: alcohols, polyols, polyoxyalkylenes, polyvinyl alcohols, polyvinyl-pyrrolidones, poly(2-methyl-2-oxazoline), a sulfate-containing moiety, a sulfonate -containing moiety, a carboxylate-containing moiety, a phosphate-containing moiety, an amine-containing moiety, a polyester-containing moiety, a polyether-containing moiety, a polyethylene glycol (PEG)-containing moiety; preferably it consists of a polyethylene glycol (PEG) polymer or a polyethylene glycol (PEG) polymer-containing moiety, comprising from 30 to 60 ethoxy monomers.

**6.** Ferrocenyl-derivative compound according to any of the preceding claims; wherein the hydrophobic moiety is fluorinated or perfluorinated.

**7.** Ferrocenyl-derivative compound according to any of claims 1 to 4; which is of formula (IIa) or (IIb) or (IIc) or (IId) or (IIe) or (IIf) or (IIg) or (IIh) or (IIi) or (IIj) or (IIa) or (IIk) or (IIl) or (IIm):

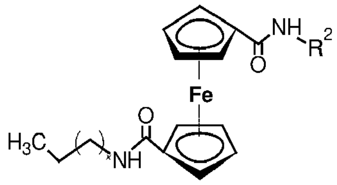
(IIa);
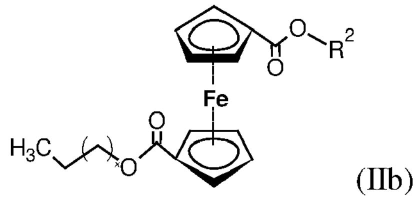
(IIb);

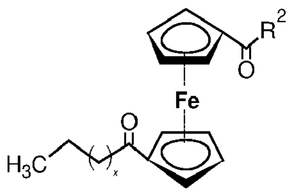
(IIc);
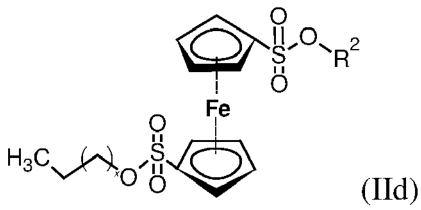
(IId);

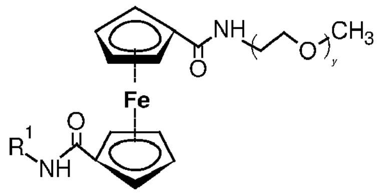
(IIe);
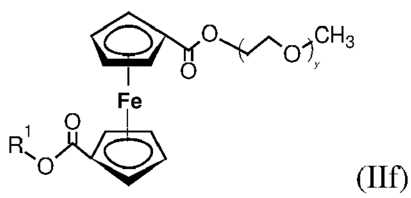
(IIf);

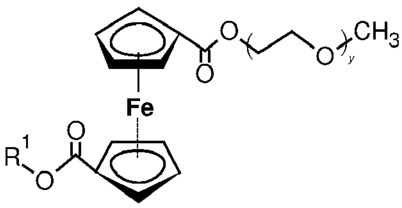
(IIg)
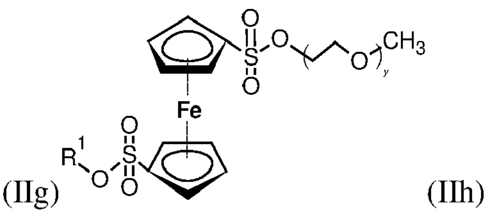
(IIh);

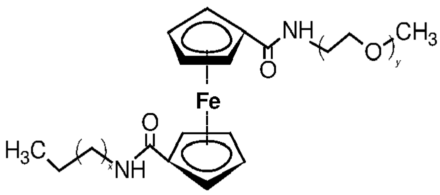
(IIi);
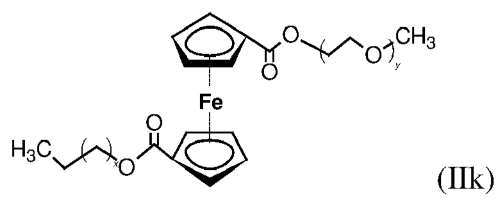
(IIk);

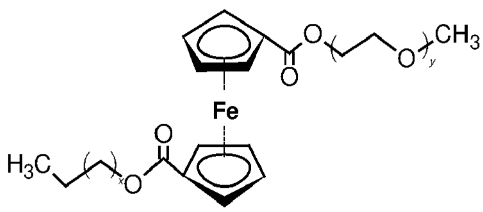
(IIl)
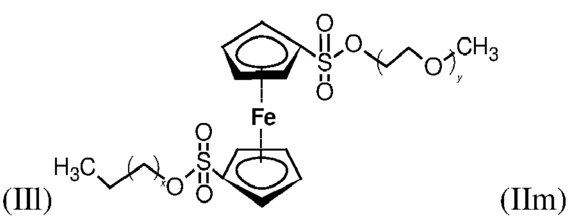
(IIm);

wherein $R^1$ is a **hydrophobic moiety** consisting of a substituted or unsubstituted, saturated or unsaturated, aliphatic $C_4$-$C_{36}$ chain-containing moiety;
wherein $R^2$ is a **hydrophilic moiety;**
wherein x is equal or superior to 2, in particular ranges from 2 to 34 ;
wherein y ranges from 8 to 100.

**8.** Biocompatible micelle composition, **characterized in that** the micelle comprises one or more ferrocenyl-derivative

compound(s) according to any of the preceding claims 1 to 7.

**9.** Biocompatible micelle composition according to claim 8, the micelle having an average hydrodynamic diameter equal or inferior to 40 nm.

**10.** Biocompatible micelle composition according to claim 8 or 9, the micelle comprising at least one long wavelength absorbing photosensitizer, the said photosensitizer having an absorption band equal or superior to 620 nm, in particular ranging from 620 nm to 1200 nm; most preferably selected from a group consisting of: phenothiazines, porphyrins, chlorins, bacteriochlorins, cyanines, phtalocyanines, and derivatives thereof.

**11.** Biocompatible micelle composition according to claim 8 to 10, or ferrocenyl-derivative compound according to any claim 1 to 7; for use as a medicament or for use in a method of diagnosis.

**12.** Pharmaceutical composition comprising a biocompatible micelle according to any of claim 8 to 11, or a ferrocenyl-derivative compound according to any of claim 1 to 7.

**13.** A kit comprising:

- one or more ferrocenyl-derivative compound(s) according to any claim 1 to 7 or a biocompatible micelle composition according to claim 8 to 11;
- one or more photosensitizer(s) having an absorption band equal or superior to 620 nm.

**14.** A method for preparing a ferrocenyl-derivative compound according to any of claim 1 to 7, comprising the steps of:

a) providing a precursor ferrocenyl-derivative compound comprising two cyclopentadienyl rings, each of the said rings being substituted with a reactive group;
b) bringing the precursor ferrocenyl compound derivative into contact with (i) a **hydrophobic moiety** consisting of an aliphatic chain-containing moiety, the aliphatic chain having at least 4 carbon atoms, and (ii) a **hydrophilic moiety,** said hydrophilic moiety being different from -OH, -C(=O)H or - C(=O)OH;
c) recovering the ferrocenyl-derivative compound.

**15.** A method for preparing a biocompatible micelle composition according to any one of claim 8 to 11, comprising a step of:

a) providing a composition comprising ferrocenyl-derivative compounds according to any claim 1 to 7, and optionally a wavelength absorbing photosensitizer having an absorption band equal or superior to 620 nm ;
b) ultra-sonicating the composition of step a);

thereby preparing the micelle composition.

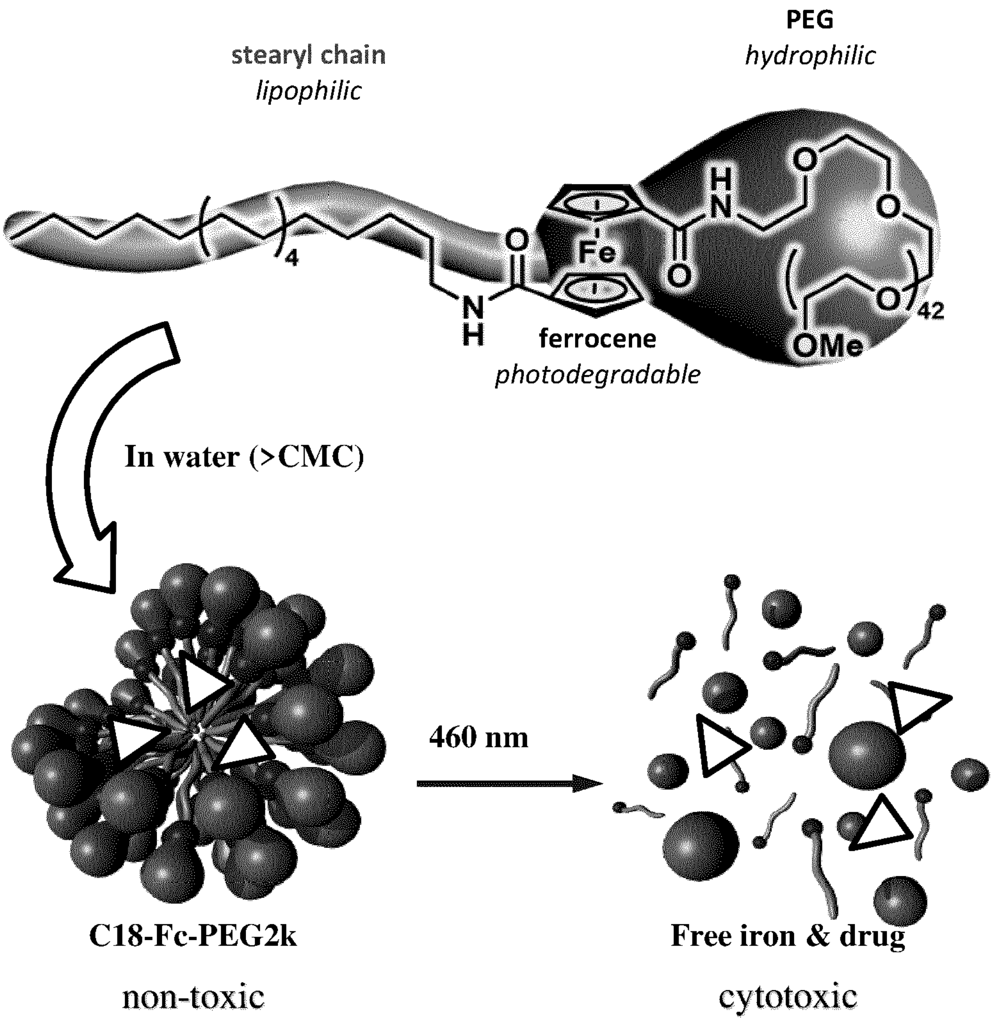
stearyl chain
lipophilic
PEG
hydrophilic
ferrocene
photodegradable
In water (>CMC)
460 nm
C18-Fc-PEG2k
non-toxic
Free iron & drug
cytotoxic

FIGURE 1

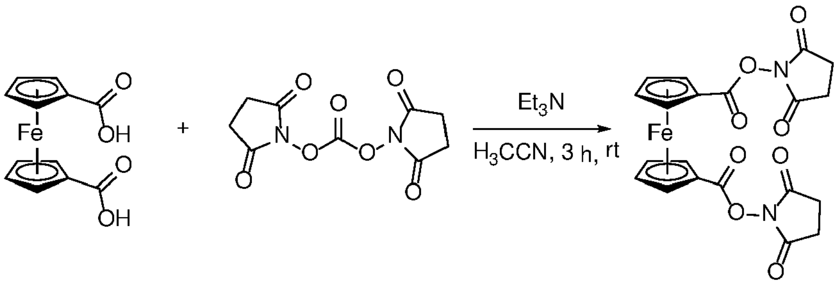


**(1)**

**60%**

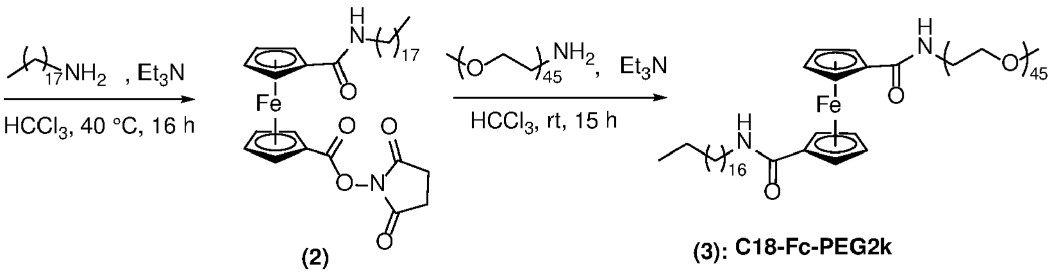


**(2)**

**32%**

**(3): C18-Fc-PEG2k**

**78%**

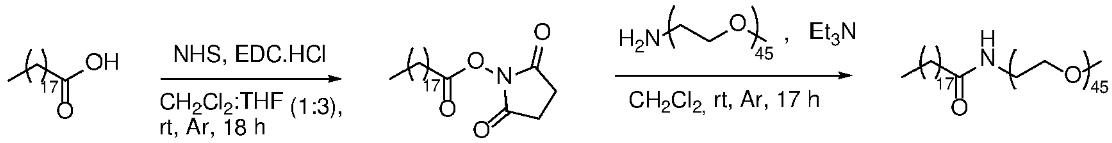


**(4)**

**78%**

**(5): C18-PEG2k**

**61%**

<u>**FIGURE 2**</u>

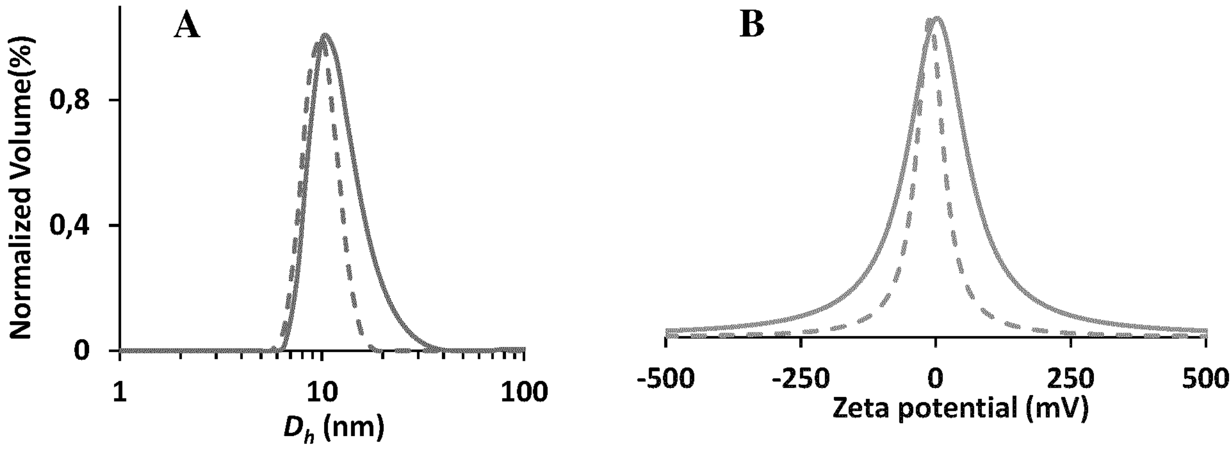


FIGURE 3

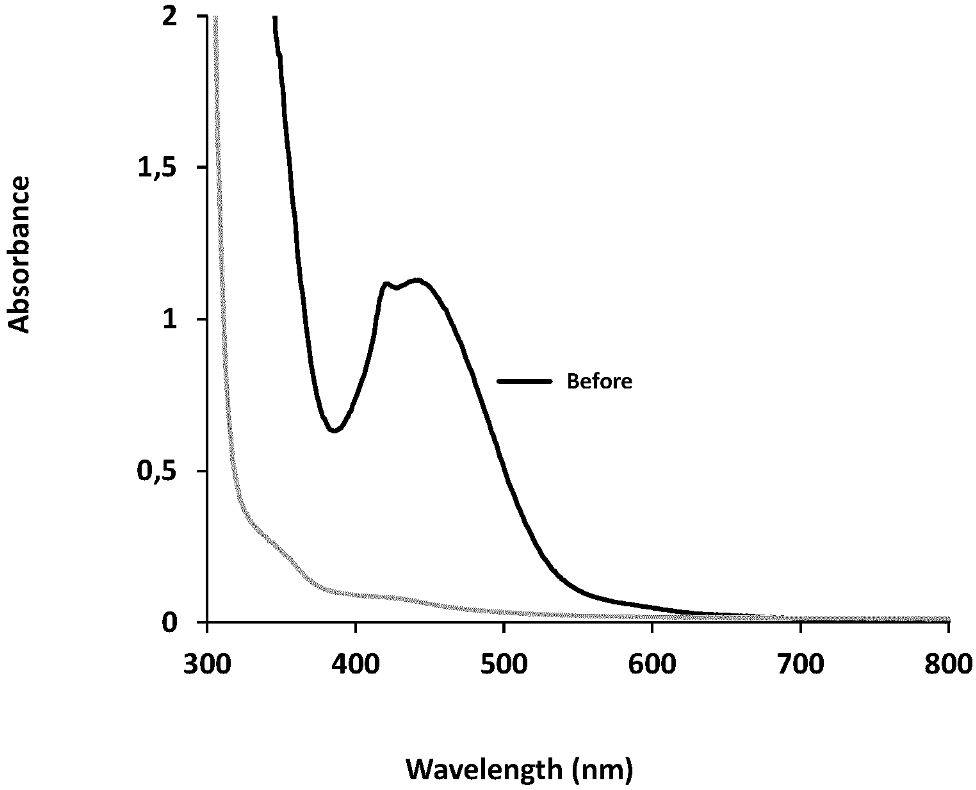


FIGURE 4

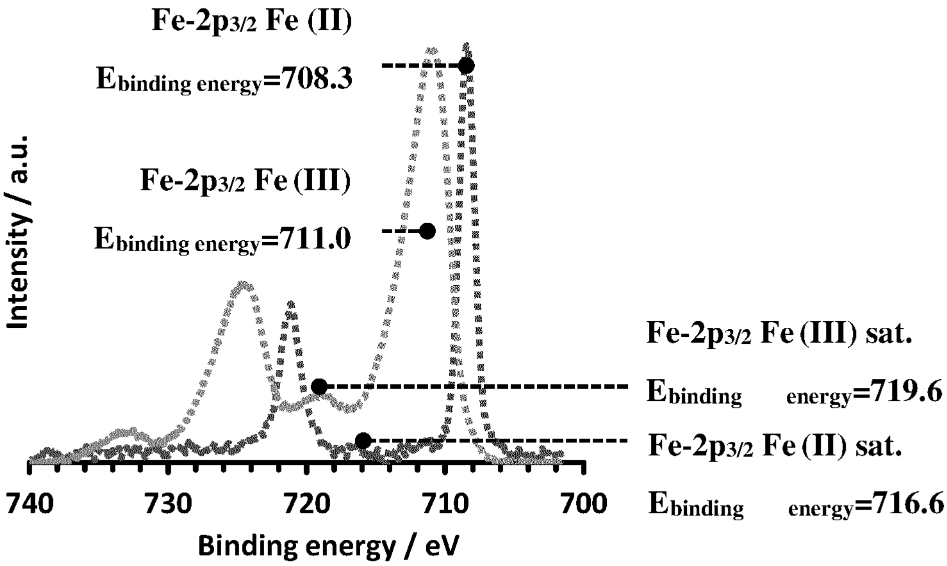


FIGURE 5

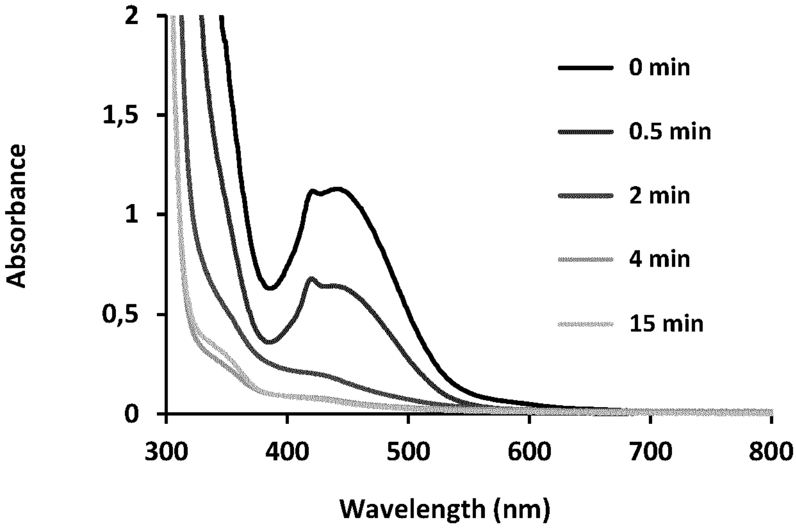


FIGURE 6

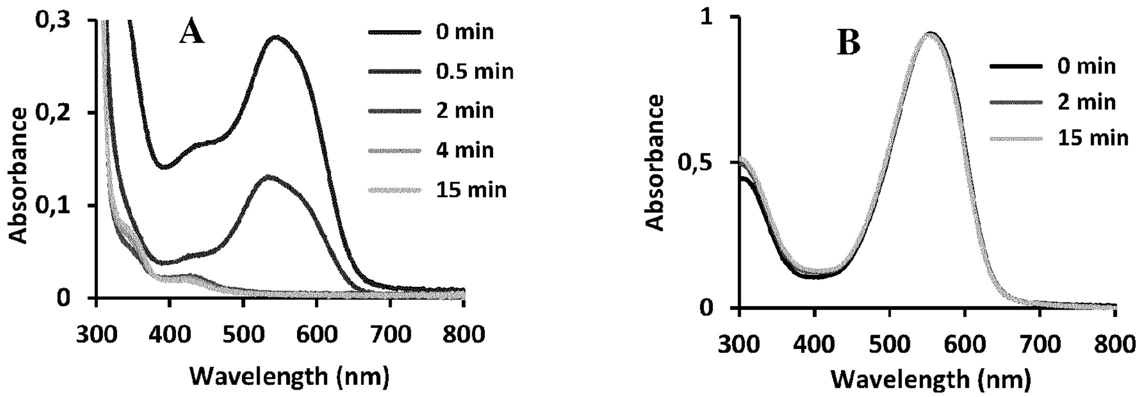


FIGURE 7

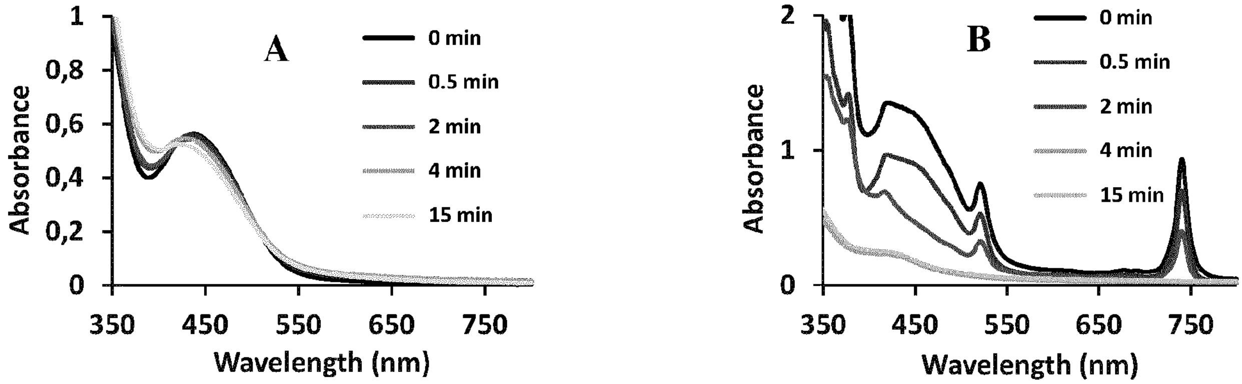


FIGURE 8

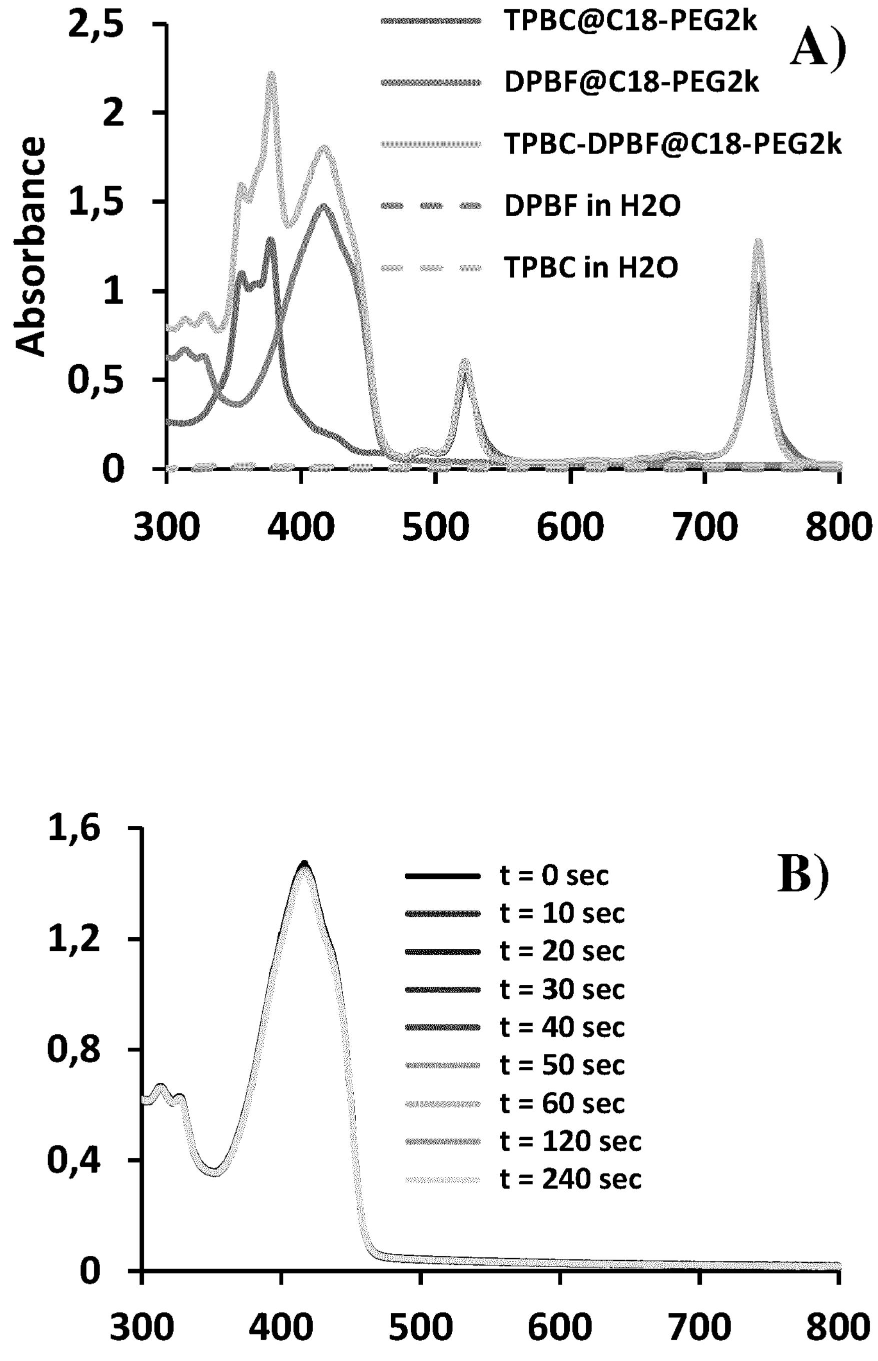
A)
Absorbance
2,5
2
1,5
1
0,5
0
300
400
500
600
700
800
TPBC@C18-PEG2k
DPBF@C18-PEG2k
TPBC-DPBF@C18-PEG2k
DPBF in H2O
TPBC in H2O
B)
1,6
1,2
0,8
0,4
0
300
400
500
600
700
800
t = 0 sec
t = 10 sec
t = 20 sec
t = 30 sec
t = 40 sec
t = 50 sec
t = 60 sec
t = 120 sec
t = 240 sec

FIGURE 9A, B

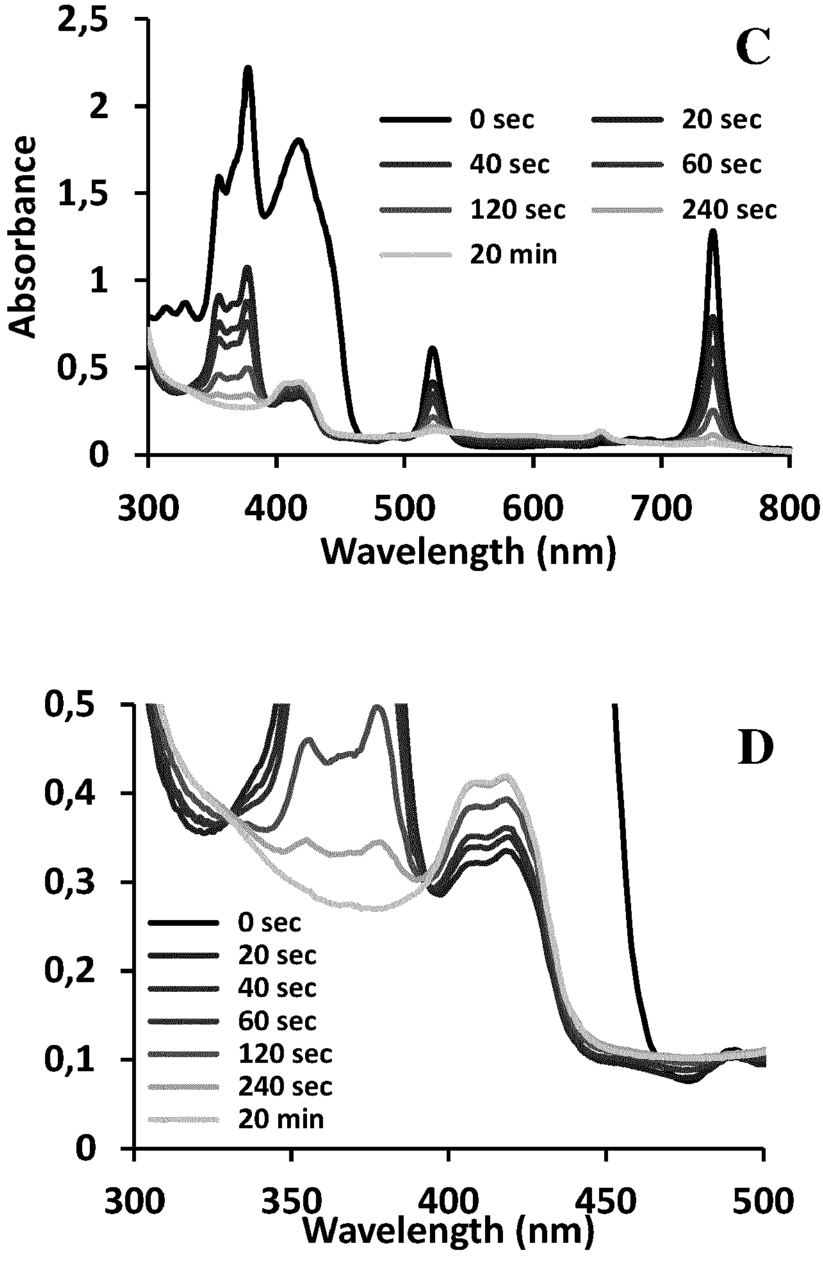
C
2,5
2
1,5
1
0,5
0
Absorbance
0 sec
20 sec
40 sec
60 sec
120 sec
240 sec
20 min
300
400
500
600
700
800
Wavelength (nm)
D
0,5
0,4
0,3
0,2
0,1
0
0 sec
20 sec
40 sec
60 sec
120 sec
240 sec
20 min
300
350
400
450
500
Wavelength (nm)

FIGURE 9C, D

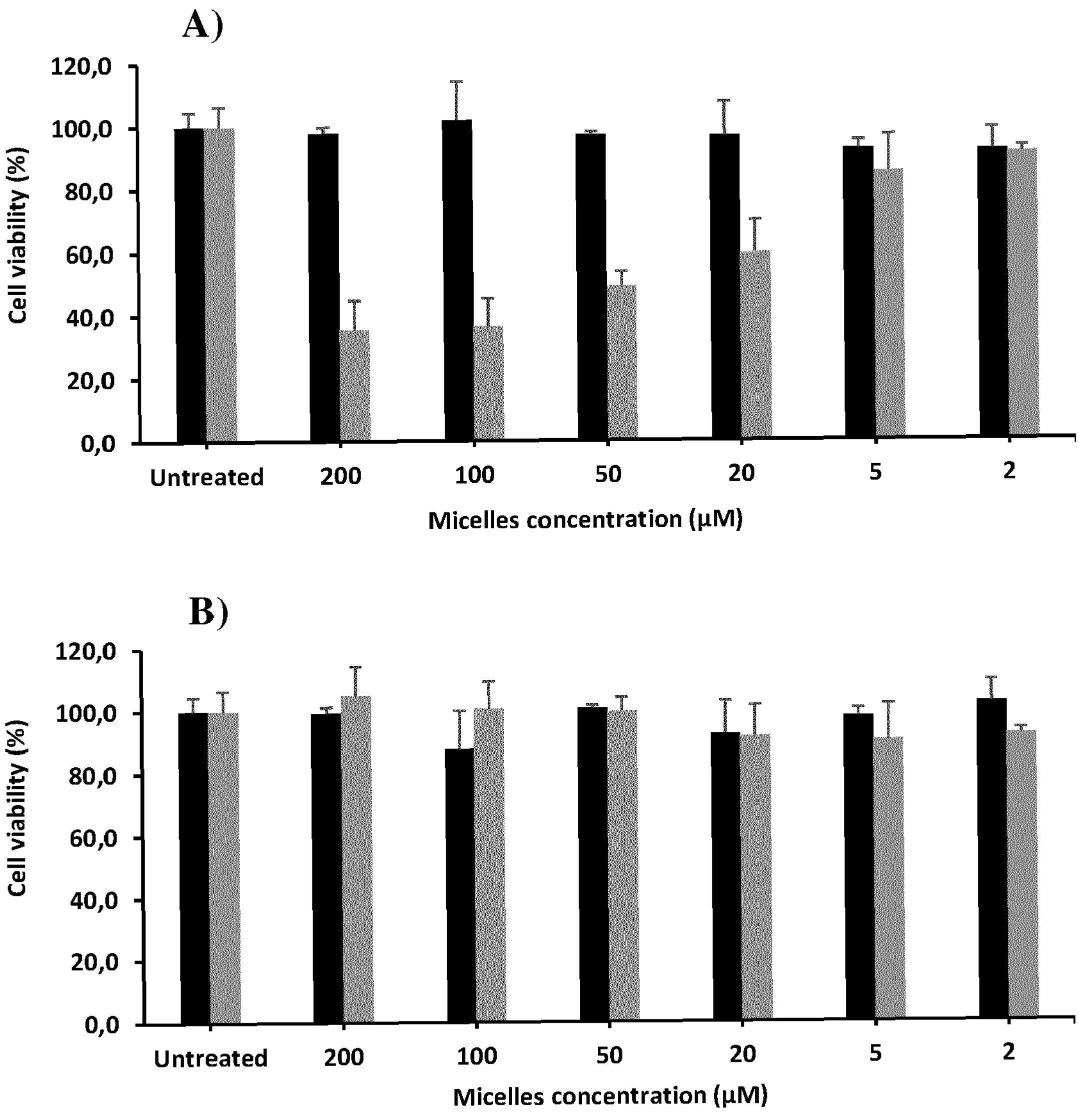
A)
120,0
100,0
80,0
60,0
40,0
20,0
0,0
Cell viability (%)
Untreated
200
100
50
20
5
2
Micelles concentration (µM)
B)
120,0
100,0
80,0
60,0
40,0
20,0
0,0
Cell viability (%)
Untreated
200
100
50
20
5
2
Micelles concentration (µM)

FIGURE 10

Europäisches Patentamt
European Patent Office
Office européen des brevets

# EUROPEAN SEARCH REPORT

Application Number
EP 23 30 5409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 2005 255582 A (JAPAN SCIENCE & TECH AGENCY) 22 September 2005 (2005-09-22) * Figure 1: release of functional molecule due to irradiation with light (paragraph [16]); claim 5: urfactant micelles with porcelain or phthalocyanine-containing photocatalytic sites that accelerate photodegradation of ferrocene in the molecule * ----- | 1-15 | INV. C07F17/02 A61P35/00 A61K9/107 A61K41/00 |
| A | QIN YI ET AL: "Self-Delivered Supramolecular Nanomedicine with Transformable Shape for Ferrocene-Amplified Photodynamic Therapy of Breast Cancer and Bone Metastases", ADVANCED FUNCTIONAL MATERIALS, vol. 31, no. 42, 1 October 2021 (2021-10-01), page 2104645, XP093076751, DE ISSN: 1616-301X, DOI: 10.1002/adfm.202104645 Retrieved from the Internet: URL:https://onlinelibrary.wiley.com/doi/full-xml/10.1002/adfm.202104645> * Ferrocenyl Fc-peptide-PEG deriviative that self-assembles with cyclodextrin derivatives Ce6-CD to form inclusion complex. The inclusion complex forms micelles which are used for photodynamic therapy of cancerous cells: see scheme 1 * ----- -/-- | 1-15 | TECHNICAL FIELDS SEARCHED (IPC) C07F A61P A61K |

The present search report has been drawn up for all claims

| Place of search | Date of completion of the search | Examiner |
|---|---|---|
| The Hague | 1 September 2023 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

Europäisches Patentamt
European Patent Office
Office européen des brevets

# EUROPEAN SEARCH REPORT

Application Number
EP 23 30 5409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YONGBIN HAN ET AL: "A Biocompatible Surfactant with Folded Hydrophilic Head Group: Enhancing the Stability of Self-Inclusion Complexes of Ferrocenyl in a &bgr;-Cyclodextrin Unit by Bond Rigidity", JOURNAL OF THE AMERICAN CHEMICAL SOCIETY, vol. 128, no. 42, 1 October 2006 (2006-10-01), pages 13913-13920, XP055035760, ISSN: 0002-7863, DOI: 10.1021/ja064591q | 1-3,5,14 | |
| A | * compound 1 in scheme 1 on page 13913; amphiphilic behaviour of compound: see whole document * | 4,6-13, 15 | |
| | ----- | | |
| X | BITTER STEFAN ET AL: "Organometallic, Nonclassical Surfactant with Gemini Design Comprising [pi]-Conjugated Constituents Ready for Modification", ACS OMEGA, vol. 3, no. 8, 31 August 2018 (2018-08-31), pages 8854-8864, XP093078207, US ISSN: 2470-1343, DOI: 10.1021/acsomega.8b01405 Retrieved from the Internet: URL:http://pubs.acs.org/doi/pdf/10.1021/acsomega.8b01405> * compounds such as FcNMe3TIPS (6) in scheme 1, comound (5) in scheme 2, compound (10) in scheme 3 * * use of compound as surfactant * | 1,3,5 | |
| | ----- | | |
| | -/-- | | |

TECHNICAL FIELDS SEARCHED (IPC)

The present search report has been drawn up for all claims

| Place of search | Date of completion of the search | Examiner |
|---|---|---|
| The Hague | 1 September 2023 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1
EPO FORM 1503 03.82 (P04C01)

Europäisches Patentamt
European Patent Office
Office européen des brevets

# EUROPEAN SEARCH REPORT

Application Number
EP 23 30 5409

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BITTER STEFAN ET AL: "Voltammetry as a Tool to Monitor the Aggregation Behavior of a Zwitterionic Ferrocene Surfactant", LANGMUIR, vol. 37, no. 14, 13 April 2021 (2021-04-13), pages 4183-4191, XP093078217, US ISSN: 0743-7463, DOI: 10.1021/acs.langmuir.1c00049 * compound FnNMe2SO3Heptene (1) in figure 1; use of compound in forming micelles: see figure 8; * * the micelles have a diameter of 4-120 nm: page 4185, left column, last paragraph * | 1,3,5,8, 9,12,14 | |
| X | DESCHENAUX ROBERT ET AL: "Organized Molecular Films from New Ferrocene-Containing Molecular Units", LANGMUIR, vol. 13, no. 8, 1 April 1997 (1997-04-01), pages 2363-2372, XP093078226, US ISSN: 0743-7463, DOI: 10.1021/la961006y * compound 13 * | 1-5,7 | TECHNICAL FIELDS SEARCHED (IPC) |
| X | WO 00/04018 A1 (KNOELL HANS FORSCHUNG EV [DE]; UNIV SCHILLER JENA [DE] ET AL.) 27 January 2000 (2000-01-27) * compounds 38-43, 47-49, pages 6-7 * | 1-7 | |

The present search report has been drawn up for all claims

1

| Place of search | Date of completion of the search | Examiner |
|---|---|---|
| The Hague | 1 September 2023 | Lange, Tim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 23 30 5409

This annex lists the patent family members relating to the patent documents cited in the above-mentioned European search report. The members are as contained in the European Patent Office EDP file on
The European Patent Office is in no way liable for these particulars which are merely given for the purpose of information.

01-09-2023

| Patent document cited in search report | Publication date | Patent family member(s) | Publication date |
|---|---|---|---|
| JP 2005255582 A | 22-09-2005 | NONE | |
| WO 0004018 A1 | 27-01-2000 | CA 2337881 A1 | 27-01-2000 |
| | | DE 19832191 A1 | 27-01-2000 |
| | | EP 1112271 A1 | 04-07-2001 |
| | | JP 2002520411 A | 09-07-2002 |
| | | US 6358967 B1 | 19-03-2002 |
| | | WO 0004018 A1 | 27-01-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ANILKUMAR et al.** Nanometric Micelles with Photo-Triggered Cytotoxicity. *Adv. Funct. Mater.,* 2014, vol. 24, 5246-5252
- **MAO et al.** Delivery of Doxorubicin from Hyaluronic Acid-Modified Glutathione-Responsive Ferrocene Micelles for Combination Cancer Therapy. *Mol. Pharmaceutics,* 2019, vol. 16, 987-994
- **WEI et al.** Preparation of Novel Ferrocene-Based Shell Cross-Linked Thermoresponsive Hybrid Micelles with Antitumour Efficacy. *J. Phys.Chem. B,* 2010, vol. 114, 5309-5314
- **XIAO et al.** Amphiphilic block copolymers with aldehyde and ferrocene-functionalized hydrophobic block and their redox-responsive micelles. *J. Mater. Chem.,* 2010, vol. 20, 8375-8381
- **YAMAGUCHI et al.** Efficient Photodissociation of Anions from Benzoyl-Functionalized Ferrocene Complexes. *Inorg. Chem.,* 1999, vol. 38, 4861-4867